# EUROPEAN PATENT APPLICATION

(11) **EP 4 269 613 A1**
(43) Date of publication of application: **01.11.2023**
(21) Application number: 20966203.0
(22) Date of filing: 24.12.2020
(51) Int. Cl.: C12Q 1/686, C12Q 1/6886, G06N 3/02, G06N 3/08, G16H 50/70

(54) **IN-VITRO METHOD FOR DIAGNOSING AND PREDICTING THE AGGRESSIVENESS OF THYROID CANCER, AND THE PRECISION SURGERY OPTIONS AND TYPE TO BE USED TO REMOVE A TUMOUR FROM A PATIENT; KIT; REAGENTS FORMING THE KIT; AND USE OF THE REAGENTS AND USE OF MOLECULAR MARKERS AS PART OF THE METHOD**

(71) Applicant: Pontificia Universidad Católica De Chile, 8331150 Santiago (CL)
(72) Inventor: GONZÁLEZ DÍAZ, Hernán Eugenio, Las Condes, Santiago (CL); URRA GAMBOA, María Soledad, Providencia, Santiago (CL); VARGAS SALAS, Sergio Sebastián, Las Condes, Santiago (CL); CÓRDOVA RIQUELME, Francisco Luciano, Santiago Centro, Santiago (CL); MARTÍNEZ SOLÍS, José Rodrigo Waldemar, Las Condes, Santiago (CL); MUÑOZ MUÑOZ, Estefanía del Carmen, Estación Central, Santiago (CL)
(74) Representative: Clarke, Modet y Cía., S.L.
(86) International application number: PCT/CL2020/050194
(87) International publication number: WO 2022/133621

(57) **Abstract**

An *in vitro* method and kit for the diagnosis and prediction of thyroid cancer aggressiveness and the possibilities and type of precision surgery for tumor removal in a subject is presented. As a part of the scope reagents for forming the kit and the use of these reagents as a part of the kit are provided.

Use of predictive markers of thyroid cancer aggression and possibilities and type of precision surgery for tumor removal in a subject is also provided.

## Description

### FIELD OF THE INVENTION

The present invention is directed to an *in vitro* method and its kits for predicting the aggressiveness of papillary thyroid cancer and guiding the most recommended surgical plan for the treatment of the tumor in a subject. The present invention describes an *in vitro* method using a molecular classifier to predict the aggressiveness (metastatic potential) of papillary thyroid cancer, allowing the surgeon to recommend the best surgical option for treating the tumor in a subject.

### DESCRIPTION OF THE PRIOR ART

Improvements in high-resolution ultrasound technologies and the increased availability and use of Fine Needle Aspiration Biopsy (FNAP) have facilitated the management and diagnosis of thyroid nodules. Because of this, a larger number of FNAPs are being performed to determine whether thyroid nodules are malignant or not (Sosa JA, et al., 2013). In the United States, approximately 350,000 FNAPs are performed each year, of which 15% are reported as cancer (Faquin WC. et al., 2011).

Orthologic diagnosis of thyroid cancer by FNAP allows cancer to be classified into 4 main types: papillary thyroid carcinoma (the most common thyroid malignancy), follicular thyroid carcinoma, medullary thyroid carcinoma and anaplastic thyroid carcinoma (Quangt. et al., 2015).

It has been described that, after an orthological diagnosis of thyroid cancer, it is important to perform preoperative staging by imaging, as it can alter the prognosis and course of treatment of the patient. To help identify potential lymphatic metastases, a preoperative ultrasonographic staging of the neck is recommended to assess the contralateral lobe and cervical lymph nodes for all patients who will undergo malignant thyroidectomy. Despite this clinical recommendation, ultrasound of the neck only identifies 50% of the lymph nodes found during surgery (Haugen et al., 2016).

As for thyroid cancer staging, the American Joint Cancer Committee (AJCC) has designated thyroid cancer staging by the Tumor, Node, Metastasis (TNM) classification system. This system is the most widely used for predicting the prognosis for thyroid cancer. However, this system focuses its analysis on patient survival rather than disease recurrence so it is not a sufficient methodology to predict recurrence, in particular, in patients with early-stage thyroid cancer.

From the staging of the patient, the treatment strategy of the patient is defined. Treatment options for thyroid cancer include surgery, radioactive iodine therapy (1311), and targeted molecular therapies with various tyrosine kinase inhibitors (TKIs).

The National Cancer Institute has defined treatment recommendations according to the stage of progress (National Cancer Institute). Surgery is presented as a standard treatment option, being considered for papillary and follicular thyroid cancer in case it is localized or metastatic, in recurrent papillary and follicular thyroid cancer and in anaplastic thyroid cancer (National Cancer Institute. Thyroid cancer treatment (PDQ). http://cancer.gov/cancertopics/pdq/treatment/thyroid/HealthProfessi onal.).

In the case of primary tumors, the surgical treatment option includes hemithyroidectomy and total thyroidectomy (removal of all visible thyroid tissue), making the selection of partial or total removal according to the size of the tumor. (Quang et al., 2015). In short, there are several variables that influence the diagnostic decision and particularly the decision of the type of surgery for the tumor removal.

Thyroid cancer is primarily diagnosed through orthology analysis. While this diagnostic strategy is the standard clinical protocol, it has been observed to have limitations such as difficulty differentiating between papillary and follicular cancer variants. In addition, this type of technique does not confer any therapeutic prognostic or predictive information. From this problem, immunohistochemical markers were worked diagnostically, however, few have the ability to determine the metastatic potential or prognosis of thyroid carcinoma (Sethi K. et al., 2010).

Different scientific articles and patent documents relating to the analysis of biomarkers for the prediction of the clinical diagnosis of patients with thyroid carcinoma have been described. Xing M. et al., for example, present a study describing novel techniques for the detection of thyroid nodules and cancer using molecular markers. Mention is made of the analysis of mutations such as RET-PTC, RAS and BRAF (V600E); galectin 3 (Xing M. et al., 2013). Additionally, the document described by González et al., in 2017, describes a gene classification prototype, which through *in vitro* diagnosis allows the characterization of nodules with indeterminate cytology.

The use of the qPCR technique and microarrangements to assess different thyroid cancer samples has also been described having markers associated with mitochondrial function and thyroid tumorigenesis as an approach (Jacques C, 2013). Other authors have identified microRNAs for the prognosis of papillary thyroid cancer (Qiu J. et al., 2018). In particular, we present the study of the relationship of the miR-146a and miR-146b levels of expression with the appearance, proliferation and prognosis of papillary thyroid carcinoma where it was detected that the miR-146a and miR-146b levels of expression can affect the proliferation and cell migration of cancer.

WO2012160519 presents a methodology for the detection of the levels of expression of one or more gene products, including the detection and analysis of the chemokine receptor CCR3. In these documents, the presence and/or level of thyroid cancer aggressiveness is determined from a sample of thyroid tissue.

On the other hand, WO2016201555, WO2012170711, US2007099209, WO2013022995, present methods based on the detection of levels of expression of one or more genes, including molecular markers such as PTEN, BRAF, CTLA4, VEGFC, among others. The approach of these documents is to obtain information regarding the presence or absence of cancer and aggressiveness thereof. Additionally, US10260103B2 presents diagnostic assays, *in vitro* methods and kits that allow identifying thyroid cancer in a biological sample, by fine needle aspiration sampling and analysis of the levels of expression of groups of genes comprising the group selected from the genes CXCR3, CCR3, CXCL10, CK19, TIMP-1, CLDN-1, CAR, XB-130, HO-1 and CCR7.

Other documents such as WO2013022995, WO2012174282 and LIS2011160290, include determining the prognosis of a thyroid cancer subject by analyzing markers such as miR-16, miR-146b, miR-155, RNU6B, among others.

There are also kits and services on the market based on the analysis of a classification model for the diagnostic prediction of thyroid cancer. In the United States, for example, the genomic classification test called ThyroSeq^{®} is a test for the preoperative assessment of thyroid nodules with indeterminate cytology, which offers an assessment of the probability of cancer in a given nodule. Although the group that offers the ThyroSeq v3 Test indicates that it provides prognostic information that helps determine what type of surgery to perform, so far there is no study that shows that Thyroseq v3 predicts an aggressiveness condition or that its use induces clinically relevant behavior change. This technology includes various versions, ThyroSeq v2 and ThyroSeq v3 which incorporate an increasingly complete panel of genes to be analyzed (Thyroseq^{®}, Thyroid Genomic Classifier). This technology is based on next-generation sequencing of DNA and RNA, a highly complex and expensive technique, which is not available in all countries of the world on a massive scale let alone for use in clinical diagnosis.

Another diagnostic test based on the genetic analysis of thyroid cancer is the Afirma^{®} genomic classification test. This test preoperatively assesses the probability or suspicion of cancer in thyroid nodules with indeterminate cytology. To this diagnostic test, the company that develops this technology (Veracyte) has incorporated the use of Afirma^{®} Xpression Atlas (XA), a test that suggests surgeons adapt the strategy of surgery or treatment options for patients whose thyroid nodules are cancerous or suspected of cancer, from the same FNAP samples used in the Afirma^{®} test. However, Affirma^{®} XA lacks clinical assay that validate its clinical utility. On the other hand, from a technical point of view, Afirma^{®} relies on complete RNA transcriptome sequencing and Afirma^{®} XA, on the detection of genomic alterations (DNA variants and RNA fusions), using the next generation sequencing platform, complex and high cost technology that does not allow the use of these molecular tests globally.

There are other tests developed that are based on mutational analyses of known biomarkers accepted by the American Thyroid Association (Quest Diagnostics and Asuragen). However, this analysis needs to be validated by clinical assays.

In general, existing methods so far require the analysis of a large number of biomarkers, tests and prediction services require analyses that must be performed in centralized service clinical laboratories and require the sending of a sample for analysis. Therefore, a diagnostic method that allows to identify with high accuracy which patients have a biological potentiality of greater aggressiveness and require an intensification of treatment plan prior to surgery has not yet been described and validated.

### DETAILED DESCRIPTION OF THE INVENTION

The present invention provides an *in vitro* method and kits for predicting the aggressiveness (metastatic potential) of papillary thyroid cancer and more accurately indicating the type of surgery that must be performed to perform a surgical treatment with a higher probability of definitive cure.

The present invention is based on the quantification of the expression of at least one or more genes in a FNAP sample from a patient with papillary thyroid cancer, which combined in a specific way by a classifier developed by neural networks allow to predict with high accuracy the metastatic potential of the tumor. The present invention is based on the quantification of the levels of expression of at least one or more genes, corresponding to BRAF, CD80, CTLA4, PTEN, VEGFC, miR-16, miR-146b, miR-155, miR-181d, RNU6B and RET.

The combinations of genes included in this invention provide significantly improved diagnostic and classification results compared to existing and available methods. The analysis by the algorithm of the expression of at least one or more of the genetic markers obtained from FNAP samples included in the present invention, yields unexpectedly high results and far superior to the predictive capacity of the genes individually or by the mere additive combination of the markers or other markers previously described. In other words, the measurement of one or more, two or more, three or more, or the set of genes of the present invention, with subsequent analysis by a step sorting algorithm, is able to predict with high diagnostic efficiency that patients with papillary thyroid cancer have an aggressive molecular profile (high metastatic potential).

As opposed to the available genetic panels (ThyroSeq V3 and Affirma^{®} GSC), which use hundreds of markers, the present invention only uses 11 markers, allowing it to be packaged in a kit ("in *vitro* diagnosis" or IVD) that may be distributed in pathology laboratories globally, expanding the availability of the diagnostic test and reducing the costs of prognostic assessment in patients with papillary thyroid cancer.

The development of the method proposed in the present invention includes the analysis of gene expression data of one or more genes through 3 steps: 1) a data pre-processing step, 2) a data processing step and 3) a classification step.

In the data pre-processing step various techniques are used to clean and transform the raw data into a useful or suitable format so that they are then used in the processing step efficiently. Raw gene expression data were prepared by various techniques to clean or transform them into a useful format for the processing step. In this first step, the generation of ratios or relationships, expansion of characteristics and reduction of dimensions were included. The latter was done with principal component analysis (PCA) that simplifies the complexity of data with multiple dimensions, while preserving trends and patterns. This analysis allows the reduction of the dimensions, that is, to reduce the number of random variables treated.

For the second data analysis step defined as data processing step, multi-layer perceptron (MLP) artificial neural network was used as a strategy to construct a classification model or algorithm. To define this model, training and cross-validation assays were performed for perceptrons with different combinations of model or algorithm hyperparameters. The hyperparameters considered were epoch, learning rates, dropouts, rho and epsilon. Of all the randomized data, 50% of them were used for the training phase and 50% for the cross-validation step. The best network with accuracy > 0.80 was chosen for the training and cross-validation cohorts.

The third step corresponds to the classification in which the scores obtained from the MLP classification algorithm or model are integrated to assess the diagnostic efficacy of the Thyroid Metastatic Classifier (CMT). According to the analysis of principal components of the independent variables included in the Prediction Classifier model, the use of the first 11 principal components that explain 99.3% of the variance between the variables was defined. This analysis then allows to establish the components that are sufficient to explain the behavior of the data included in the CMT model. A receiver operating characteristics (ROC) curve was developed to define the diagnostic potential of the genetic signature, reflecting the strength in predicting metastatic potential. From this the area under the curve (AUC) was defined. The genetic signature of the classifier was constructed after different iterations of prediction, until the most sensitive AUC was obtained.

With the definition of AUC, where the classifier used 11 genes that were integrated by the neural network, performance was determined to predict lymph node metastases. According to the results, the CMT predicts with high efficiency the cases with high metastatic potential in FNAP samples from patients with papillary thyroid cancer. The cut-off for classifying samples as metastatic (M-PTC) or non-metastatic (NM-PTC) was 0.20.

Then, a prediction method based on a Thyroid Metastatic Classifier with the best diagnostic performance for lymph node metastasis is provided. In the training cohort, the diagnostic efficacy of the classifier reached an AUC of 0.82 [Cl 0.73 - 0.92], a sensitivity of 79% [CI 61-92] and a specificity of 76% [Cl 61-88], VPP and VPN were 70% [Cl 56 - 80%] and 85% [Cl 73 - 92%], respectively. In the statistical validation cohort, the TPC reproduced its efficacy, showing an AUC of 0.84 [Cl 0.76 - 0.95], a sensitivity of 72% [Cl 70 - 96%] and a specificity of 87% [CI 61-88%]. VPP and VPN were 79% [Cl 59-90%] and 82% [Cl 69-90%], respectively.

The positive and negative predictive values of CMT were estimated according to Bayes' theorem. The classifier is expected to reach a positive predictive value of 60-79% and a negative predictive value of 94-82% in a range of prevalence of lymph node metastasis of 25-40%.

The *in vitro* method proposed in the present invention allows identifying the cancerous thyroid tissue sample as metastatic or non-metastatic with a sensitivity greater than or equal to 69% or greater than or equal to 90%; a specificity greater than or equal to 78% or greater than or equal to 93%; a positive predictive value greater than or equal to 52% or greater than or equal to 76%; a negative predictive value greater than or equal to 55% or greater than or equal to 93%; a positive probability ratio greater than or equal to 2 or greater than or equal to 12; a positive post-test probability greater than or equal to 58% or greater than or equal to 90%; a negative probability ratio greater than or equal to 0.19 or greater than or equal to 0.58; and a negative post-test probability greater than or equal to 9% or greater than or equal to 32%.

Specific details are presented in the following description that allow understanding of the various embodiments of the invention.

### Definitions

The terms presented herein have the same meaning as is commonly used in the prior art, unless otherwise indicated. The terms used in this document are presented below:
By "about" is meant an amount, level, value, number, frequency, percentage, dimension, size, amount, weight or length ranging up to 30, 25, 20, 15, 10, 9, 8, 7, 6, 5, 4, 3, 2 or 1% at a reference amount. In any embodiment discussed in the context of a numerical value used in conjunction with the term "about," it is contemplated that this term may be omitted.

In the present invention, the term "comprise" is presented in the specification and in the set of claims and variations thereof ("comprises and "comprising ") are to be interpreted in an open and inclusive sense, i.e. as "including, but not limited to".

When the term "consisting of" is used, it is indicated that the elements listed in the sentence are mandatory and may not be present in other elements. In addition, the term "consisting essentially of" is used for any item listed after the phrase and limited to other items that do not interfere with the specific activity or action of disclosing the listed items or that do not contribute.

A "reduced," "decreased," "lower" amount corresponds to a "statistically significant" amount of decrease in values, amounts, measurements in results. The term "increased" or "improved" corresponds to a "statistically significant" amount of increase in values, amounts, measurements in results. Both the "statistically significant" increase and decrease are compared to the control values of each assay. The decrease or increase of the values obtained in the results with respect to the control may range approximately from 0.1 to 1,000, for example, a sample may increase its levels of expression with respect to the control 50-fold.

The term "obtained from" refers to a sample as such being isolated or derived from a particular source. The same applies to the term "derived from", which can refer to the source from which the sample comes.

The specific term "an embodiment" indicates particular characteristics or structure described in connection with the embodiment including at least one embodiment present in the invention, which do not necessarily correspond to the same embodiment. On the other hand, particular structures or characteristics may combine one or more embodiments. The specific term "quantification" indicates an embodiment of the present invention, which refers to the counting in numbers or the expression of magnitude through numbers or percentages. When referring to "quantification of levels of expression" it refers to the quantification of gene expression products which may be indicated as amount or percentage of proteins or mRNA.

Unless otherwise indicated in the definitions presented herein, all technical and scientific terms used have the same meaning as those of skill in the art. For the present invention, the following scientific terms will be defined below:
Coding sequence: means any nucleotide or polynucleotide sequence that contributes to the peptide or polypeptide product of a gene. In turn, the term "non-coding sequence" indicates nucleotide or polynucleotide sequences that do not contribute to the polypeptide product of a gene.

Gene: "gene" means an inheritance unit that occupies a specific place on a chromosome and consists of transcriptional and/or translational regulatory sequences and/or a coding region and/or non-translated sequences as introns.

Gene product: the term "gene product" is understood as that product resulting from the expression of a gene, which may be a protein or RNA.

Gene expression: the term "gene expression", "gene expression" refers to the cellular process that allows to transform the information encoded by nucleic acids into proteins. When the term "overexpression" or "gene overexpression" is indicated, it refers to obtaining a high number of proteins, after the transformation process from nucleic acids.

Isolated: it is understood as a material that is free from components that normally accompany this material in its native state.

MRNA or mRNA transcripts: Included herein are, but not limited to, pre-mRNA transcripts, mature mRNAs and translation and transcription of these sequences. An mRNA refers to a nucleic acid for the synthesis of which DNA has been used as a template.

cDNA: the definition of cDNA is included, which describes that a cDNA corresponds to an inverse form of DNA which comes from mRNA. A cDNA, RNA, amplified RNA, amplified DNA, etc. for purposes of this application are derived from transcription of mRNA and detection of products derived from this transcription. MRNA samples include, but are not limited to mRNA transcripts of the gene (s), reverse transcription of cDNA from the mRNA, transcription of cRNA from cDNA, amplified DNA from the genes, transcribed RNA from amplified DNA, and the like.

Amplification: When referring to the term "amplification", "sequence amplification", it refers to increasing or increasing the amount or increasing the number of copies of a gene product or gene sequences. The term "amplification" is used in molecular assays such as the Chain Polymerase Reaction or PCR. When referring to PCR assays, reference is made to that molecular assay which allows obtaining a greater number of copies of a gene product. When the present invention refers to real-time PCR or qPCR or quantitative PCR it refers to the molecular method that allows to amplify and quantify the gene product that was amplified. The real-time PCR or qPCR or quantitative PCR technique employs specific primers or primers, and specific reagents for such a reaction, which is thermostable, however, unlike conventional PCR, this method further uses a fluorophore which is identified by a fluorescence sensor that allows quantification of the amplified gene product. On the other hand, in these techniques mentioned in this invention, "reagents" are used, which comprise chemical substances that allow interaction with different components in a reaction or allow the generation of new substances. The term "polynucleotides" or "nucleic acid" is referred to which refer to the polymeric form of nucleotides of at least 10 bases in length, ribonucleotides or deoxyribonucleotides or a modified form thereof. Single-stranded and doublestranded structures of DNA and RNA are included in this term. In this invention, polynucleotide sequences may include genomic, extragenomic sequences, plasmid-encoded sequences, gene segments designed for expression, peptides or sequences designed adapted for peptide expression. Such genomic segments may be modified by the hand of man. The polynucleotides of this invention may be combined with other DNA sequences, such as, for example, promoters, polyadenylation signals, cleavage sites for restriction enzymes, among others. Thus, it is possible to use a fragment of nucleotide sequences of almost any length, preferably limited by the maximum length allowing ease of preparation and use in recombinant DNA protocols.

Polynucleotide variant: This term refers to polynucleotides that exhibit substantial sequence identity to the reference polynucleotide sequence. This term contemplates polynucleotides that are distinguished from one another by addition, deletion or substitution of at least one nucleotide.

Sequence Identity: This term is used herein to refer to the degree to which sequences are identical in nucleotide sequences per nucleotide or amino acid per amino acid as compared to another sequence.

Polypeptide and protein: refer to a polymer of amino acid residues and analogous and natural synthetic variants thereof. These terms apply to synthetic or non-synthetic amino acid polymers.

Probe: refers to a small DNA or RNA fragment, which is used in molecular biology techniques to identify the presence of DNA or RNA. The probes are characterized by having the ability to bind or hybridize with sequences or sectors of DNA or RNA sequences. Furthermore, these probes may or may not contain "fluorophores" which emit a light when this probe is attached to a DNA or RNA sequence. The term "fluorophore" refers to molecules or components thereof that are fluorescent in that they contain a functional group which absorbs a specific wavelength that it then emits in the form of light.

Subject or individual: herein a "subject" or "individual" is referred to as any animal exhibiting a symptom, or could exhibit a symptom which may be treated or diagnosed according to the invention. Included are subjects for profiling the gene products of the invention for diagnosis and other purposes. Suitable subjects or individuals (patients) include laboratory animals, farm animals, domestic animals. Mammals such as primates and humans are included.

Treatment: This term includes any desirable effect on the symptoms or pathology of a disease or condition, e.g. thyroid cancer. This term does not necessarily indicate eradication or cure of the disease or associated symptoms thereof. The treatments may be applied to subjects in need thereof.

Diagnosis: when referring to the term "diagnosis" it includes any type of recognition, analysis and assessment that is performed to determine a given situation and what its characteristics are. In medical terms, the diagnosis makes it possible to identify the appearance of diseases in an individual by observing symptoms and applying tests and analyses that allow the determination of the disease.

Prediction: the term "prediction" refers to the study of possible future scenarios through different analysis techniques. In the case of the present invention, a "prediction" performed by a diagnostic method allows determining whether or not in the future the disease being diagnosed in the individual may worsen and thereby assessing the treatment conditions for the latter.

Prognosis: when the term "forecast" is referred to, it refers to the estimation of processes or situations where uncertainty is generated. With respect to the present invention the term "prognosis" refers to the ability to report the progress of a disease in the near future, anticipating its possible evolution, recovery and effectiveness of the treatments applied in required cases.

Wild type: refers to a microorganism, gene, or gene product having the characteristics of that microorganism, gene, or gene product that is isolated from a natural source.

Differential Expression: is understood in the present invention by "differential expression", when a biological sample is indicative of cancer by exhibiting differences in the expression of one or more, two or more, or three or more of the gene products or in combination thereof, in said biological sample as compared to the expression in a normal (non-cancerous) control sample. Differential expression includes a statistically significant difference in one or more levels of expression of a gene product compared to levels of expression of the same gene product that has been determined as control. The statistically significant difference may be an increase or decrease in levels of expression, as measured by RNA levels, protein levels, protein function or any other relevant measure of gene expression indicated herein. The term "statistically significant" is used when the result obtained is unlikely to occur by chance. This result will present a level of significance that will be related to a concept of frequent statistical hypothesis testing. "Statistical significance" may be defined as the probability of making a favorable or unfavorable decision according to the hypothesis you have, this decision is determined using the "p" value, if "p" is less than the level of significance, the hypothesis is rejected. The smaller the p-value, the more significant the results. In this document, Bayes factors are also presented, which are used for the determination of statistical significance, thus, an analysis may be reflected in which the probability of making the decision to reject the null hypothesis when the null hypothesis is actually true is not greater than the established probability. With this type of analysis, decisions may be made for sets of probabilities with greater accuracy.

### Diagnostic assays.

The present invention is based on the initial identification of new molecular markers of papillary thyroid cancer, thereby allowing the classification of the degree of aggressiveness of the tumor. Therefore, the present invention presents diagnostic assays with which biological samples obtained from a subject with papillary thyroid cancer may be analyzed and the degree of tumor aggressiveness determined. In addition, the present invention allows determining the type of surgery to which the subject may be subjected in case of suffering from more aggressive papillary thyroid cancer, increasing the chances of success of this.

In various embodiments, the methods of the present invention also include the step of performing an orthological or histological analysis on a biological sample, e.g., a thyroid tissue sample obtained from the subject, e.g., for the purpose of defining a preliminary diagnosis. Orthological or histological analysis may be performed prior to, concurrent to, or subsequent to performing analysis based on the expression of gene products, as described herein. In certain embodiments, samples with a preliminary diagnosis of papillary thyroid cancer are analyzed by the methods of the present invention. In particular embodiments, the method further comprises identifying the subject at risk of aggressive thyroid cancer by orthologic or histochemical analysis of biological samples obtained from the subject, e.g., by needle or fine needle aspiration biopsy techniques.

In particular embodiments, abnormal and uncontrolled growth of cells in the thyroid may form nodules that may be benign or malignant. Benign or non-cancerous tumors include nodular hyperplasia (NHP), lymphocytic thyroiditis (LCT) and Hürthle cell adenoma (HA), follicular adenoma (FA). While malignant tumors include papillary, follicular, medullary, and anaplastic tumors, which in turn present subtypes of tumors such as follicular variant of papillary carcinoma (FVPTC), medullary thyroid carcinoma (MTC), papillary thyroid carcinoma (PTC), Hürthle cell carcinoma (HC), anaplastic thyroid carcinoma (ATC), and follicular carcinoma (FC). These cancers have metastatic potential, for example, an aggressive medullary or follicular thyroid cancer or a medullary or follicular thyroid cancer with metastatic potential. "Metastatic potential" refers to the ability or possibility for a cancer cell to move from an initial site (in this case the thyroid gland) to other sites in the body, and may develop in other organs.

In other particular embodiments of the present invention, there is provided a method for the diagnosis, identification or classification of a cancer, particularly thyroid cancer in a subject. The proposed method comprises the steps of analyzing tissue samples for the determination of levels of expression of one or more gene products and from that sample identifying whether it corresponds to a cancer sample with lymph node metastasis. Identification of expression of the gene products will indicate that the biological samples do not metastasize when these levels of expression of one or more gene products indicate low expression of these products associated with tumor aggressiveness factors. Instead, a biological sample with high degree of malignancy will be identified as such, when the expression of one or more gene products is indicative of lymph node metastasis. In particular embodiments, provided herein is a method comprising determining expression of one, two or more, three or more, gene products obtained from the biological sample of thyroid tissue. The gene products that are expressed are seen in Table 2 and wherein at least one of the gene products is expressed by the BRAF, CD80, CTLA4, PTEN, VEGFC, miR-16, miR-146b, miR-181d, RNU6B, RET gene. In certain embodiments of this invention, the method comprises performing total or partial thyroid removal surgery on the subject, depending on the degree of tumor aggressiveness determined in the biological sample with papillary thyroid cancer. In particular embodiments of the present invention, the gene product to be analyzed corresponds to RNA and the assay for analysis thereof comprises PCR, RT-PCR or qPCR, or any other assay that allows determining the amount of RNA or levels of expression, including the assays described herein. In particular embodiments, the gene product to be analyzed is a polypeptide, and the analysis assay corresponds to immunohistochemistry or any other assay that allows determination of amounts of polypeptides or levels of expression, where those presented herein are included.

In other particular embodiments, the biological sample was obtained from a subject suffering from papillary thyroid cancer. Gene products for which gene expression is determined include those described herein and may comprise one or more of these gene products, which may also be referred to as a "gene product set". This set of gene products may be used for the determination of the presence or absence of lymph node metastasis in papillary thyroid cancer. The set of gene products may be referred to as "biomarkers".

In particular embodiments, the present invention provides a method of detecting or diagnosing the presence or absence of lymph node metastasis in papillary thyroid cancer in a subject comprising determining the levels of expression of two or more, or three or more, gene products that are expressed by the genes listed in Table 2, and identifying the thyroid tissue sample as metastasized cancerous or non-metastasized cancerous by correlating the levels of expression determined by the biological sample from the subject with the presence or absence of lymph node metastasis.

In other related embodiments the present invention includes a method of treating a subject in need thereof, comprising: determining whether the subject has a cancer, particularly papillary thyroid cancer, by any of the diagnostic methods of the present invention; and performing surgery contemplating complete surgical tumor removal from the subject with cervical lymph node dissection or only complete tumor removal or a portion thereof, in the event the subject is determined to have a cancer, particularly papillary thyroid cancer.

The method of treating a subject in need thereof comprises: (i) determining whether the subject suffering from papillary thyroid cancer has lymph node metastasis by a method comprising performing an assay for determining a level of expression for one or more gene products of a biological sample of thyroid tissue and identifying whether or not this sample of thyroid tissue has lymph node metastasis or the levels of gene expression in these tissues are suspected to be indicative of a tumor aggressiveness, (ii) performing partial or total removal surgery of the cancerous tissue in the subject's thyroid and/or cervical removal of the lymph nodes, if analysis of the subject (i) indicates that the subject is likely to have papillary thyroid cancer with lymph node metastasis.

In certain embodiments, the present invention includes a method for determining whether a subject exhibits cancer with some degree of tumor aggressiveness, for example, a papillary thyroid cancer with lymph node metastasis, wherein an initial test performed on a biological sample obtained from the subject, for example, by FNAP of thyroid tissue, exhibited a cancerous result, the method comprising performing, requesting, or obtaining the results of a diagnostic assay described herein performed on a biological sample, for example, a thyroid sample, obtained from the subject. In particular embodiments, the diagnostic assay includes performing an assay to determine an level of expression for one or more gene products from a biological sample, e.g., a thyroid tissue sample; and identifying the biological sample as cancerous without lymph node metastasis wherein the level or levels of expression of the gene product indicates a lack of lymph node metastasis in the biological sample or identifying the biological sample as malignant or aggressive cancer wherein the level or levels of expression of the gene product is indicative of lymph node metastasis in the biological sample. In particular embodiments, the method comprises determining an level of expression of two or more, or three or more, gene products in the thyroid tissue sample, wherein the two or more, or three or more, gene products are expressed by one or more genes listed in Table 1 and wherein at least one of the gene products is expressed by the gene BRAF, CD80, CTLA4, PTEN, VEGFC, miR-16, miR-146b, miR-155, miR-181d, RNU6B, RET.

In particular embodiments of the present invention, in any of its methods, the correlation is performed by comparing the levels of expression of the gene products of the thyroid tissue sample and the reference levels of expression for each gene product in question. Biological samples of thyroid tissue are identified as cancerous with lymph node metastasis, if there is a significant difference in level of expression of the gene products between the thyroid tissue sample and normal control or reference levels of expression. In certain embodiments, the thyroid tissue sample is identified as cancerous with lymph node metastasis, if there is a significant difference in level of expression of two or more, three or more, or four or more gene products between the thyroid tissue sample and a normal control or reference expression of levels. Likewise, the thyroid tissue sample is identified as cancerous without lymph node metastasis, if there is no significant difference (i.e., substantial similarity) in the level of expression of the gene products between the thyroid tissue sample and normal control or reference levels of expression. In certain embodiments, the thyroid tissue sample is identified as cancerous without lymph node metastasis, if there is no significant difference in the level of expression of two or more, three or more, or four or more gene products between the thyroid tissue sample and a normal control or expression of reference levels.

In embodiments of the present invention of any of the methods described herein, the tissue sample is identified as cancerous with lymph node metastasis, if the level of expression of one or more of the genes (NM_005429.5, MIMAT0000069, MIMAT0002809, MIMAT0000646, MIMAT0002821 and NR_002752.2) is decreased, or if the level of expression of one or more of the genes (NM_005214.5) is increased in the thyroid tissue sample, as compared to normal levels of expression (levels of expression used as a reference), the decrease or increase in gene levels of expression is seen by the increase or decrease in expression of at least 3 genes. The identity of each gene corresponds to: BRAF (Gene 1), CD80 (Gene 2), CTLA4 (Gene 3), PTEN (Gene 4), VEGFC (Gene 5), miR-16 (Gene 6), miR-146b (Gene 7), miR-155 (Gene 8), miR-181d (Gene 9), RNU6B (Gene 10), RET (Gene 11). In other embodiments of any of the methods described herein, a thyroid tissue sample is identified as cancerous with lymph node metastasis, if the level of expression of one or more, two or more, three or more, or four or more gene products from any of the subsets of genes described herein is altered as described above. In particular embodiments, the gene set includes one or more genes corresponding to BRAF, CD80, CTLA4, PTEN, VEGFC, miR-16, miR-146b, miR-155, miR-181d, RNU6B, RET.

In particular embodiments, the correlation is performed by comparing the gene levels of expression of the gene products from the biological sample obtained from the subject with the reference levels using an algorithm. Such reference levels may include levels of expression of each gene product per individual from a variety of cancerous biological samples with lymph node metastasis and cancerous without lymph node metastasis.

In certain embodiments, comparison of levels of expression allows a correlation between gene levels of expression of the different samples to be determined. The biological samples used for the generation of this correlation comprise a variety of thyroid tissue samples, among which are cancerous tissue samples without indicators of tumor aggressiveness and cancerous tissue samples with indicators of tumor aggressiveness, the latter being considered cancerous with lymph node metastasis where the levels of expression of the above-mentioned genes present a significant difference in the levels of expression of the gene product with respect to the levels of expression of normal cancerous tissue, if this difference did not exist or was not a substantial difference the sample is considered to be normal.

In embodiments of the present invention, the method allows identifying the cancerous thyroid tissue sample as metastatic or non-metastatic with a sensitivity greater than or equal to 69% or greater than or equal to 90%; a specificity greater than or equal to 78% or greater than or equal to 93%; a positive predictive value greater than or equal to 52% or greater than or equal to 76%; a negative predictive value greater than or equal to 55% or greater than or equal to 93%; a positive probability ratio greater than or equal to 2 or greater than or equal to 12; a positive post-test probability greater than or equal to 58% or greater than or equal to 90%, a negative probability ratio greater than or equal to 0.19 or greater than or equal to 0.58; and a negative post-test probability greater than or equal to 9% or greater than or equal to 32%.

In particular embodiments of the invention, any of the methods or kits presented in this invention, levels of expression of two or more gene products, one or more, two or more, or three or more gene products are determined, where the genes are BRAF, CD80, CTLA4, PTEN, VEGFC, miR-16, miR-146b, miR-155, miR-181d, RNU6B, RET.

In particular embodiments, any of the foregoing gene products, two or more, three or more gene products include one or more BRAF, CD80, CTLA4, PTEN, VEGFC, miR-16, miR-146b, miR-155, miR-181d, RNU6B gene products. In particular embodiments, the gene products include one or more, or three or more of the genes mentioned in Table 2, wherein at least one of the gene products corresponds to BRAF. In particular embodiments, the gene products include one or more, or three or more of the genes mentioned in Table 2, wherein at least one of the gene products is CD80. In particular embodiments, the gene products include one or more, or three or more of the genes mentioned in Table 2, wherein at least one of the gene products is CTLA4. In particular embodiments, the gene products include one or more, or three or more of the genes mentioned in Table 2, wherein at least one of the gene products corresponds to PTEN. In particular embodiments, the gene products include one or more, or three or more of the genes mentioned in Table 2, wherein at least one of the gene products includes analysis of the VEGFC gene. In particular embodiments, the gene products include one or more, or three or more of the genes mentioned in Table 2, wherein at least one of the gene products corresponds to miR-16.

In particular embodiments, the gene products include one or more, or three or more of the genes mentioned in Table 2, wherein at least one of the gene products corresponds to miR-155. In particular embodiments, the gene products include one or more, or three or more of the genes mentioned in Table 2, wherein at least one of the gene products is miR-181d. In particular embodiments, the gene products include one or more, or three or more of the genes mentioned in Table 2, wherein at least one of the gene products is RNU6B.

Also included in the present invention are methods and equipment (kits) useful for the characterization of thyroid cancer. The term "characterization of thyroid cancer" in a subject refers to the identification of one or more properties that may be found in a cancer sample obtained from a subject suspected of this disease. For example, one type of thyroid cancer may contain specific characteristics and include determining the prognosis and survival of the subject. Types of thyroid cancer may be identified by molecular markers, where included, the gene products set forth herein.

In certain embodiments of the present invention, methods, software, and systems of biology may be employed for the diagnosis of cancer, particularly thyroid cancer. In certain embodiments, various computer program products and software may be employed for various purposes such as data management, partition design, probe design, among others.

One of ordinary skill in the art will appreciate that the general methods described herein may be readily adapted to determine the type of thyroid cancer, for example, by comparing the levels of expression of the gene products to those determined for various types of thyroid cancer. Based on the determination of the type of thyroid cancer, it may be determined to estimate the prognosis, survival and/or probability of metastasis, e.g., based on historical data or results.

### Genes and gene products

The methods described herein include assays for determining the molecular profiles of biological samples by techniques that allow the levels of expression of the gene products of the genes or sets of genes identified for carrying out the present invention to be determined. Included within the gene products are, but are not limited to mRNA and proteins expressed by such genes. Gene products (also referred to as "gene expression products") are analyzed by determining or measuring the levels of expression thereof (genes presented in Table 2, their variants or homologues in other species, or additional gene products) in accordance with the methods presented herein.

In particular embodiments, the methods used in the present invention are presented for the determination of the levels of expression of one, two, three, four, five or more genes corresponding to those presented in Table 2 of a sample of tissue, particularly thyroid tissue. In particular, gene levels of expression determined in the analysis of one or more genes or in combination thereof, are determined by analysis of the expression of the genes BRAF, CD80, CTLA4, PTEN, VEGFC, miR-16, miR-146b, miR-155, miR-181d, RNU6B, RET.

The level of expression of the gene products may be determined by any one of the available techniques depending on the gene product to be assessed. The gene products may be assessed by reagents that allow determining mRNA expression, protein expression or activation, or subsequent biological functions of the proteins that are encoded by the genes described in Table 2. In consideration of the foregoing, this document includes reagents for detecting such genes or gene products thereof.

In certain embodiments of this invention, the gene products may be determined using antibodies such as, immunohistochemistry using specific antibodies, a polypeptide array or an antibody array, immunoblotting, Western Blotting, ELISA assay, flow cytometry, immunofluorescence. In certain embodiments, mass spectrometry (MS) or other means based on molecular weight determination may be employed. In certain embodiments, techniques may be used to quantify the presence or levels of gene products, these techniques correspond to flow cytometry, immunofluorescence, *in situ hybridization,* fluorescence *in situ* hybridization (FISH).

In certain embodiments of the invention, the methods of the invention may include isolating one or more gene products from the biological sample, these gene products may comprise polypeptides, peptides, DNA, RNA. In certain embodiments, the isolation of gene products such as RNAs may be isolated by already known nucleic acid extraction techniques. RNA levels of expression are determined by known techniques, including PCR, RT-PCR and qPCR.

In certain embodiments, amplification techniques may be employed for the gene products. The term "amplification" or "nucleic acid amplification" is defined as the production of multiple copies of at least a portion of a target nucleotide sequence, under "amplification conditions" that allow amplification of the target nucleotide sequence, the result of this nucleotide sequence amplification is referred to as an "amplicon" and each amplicon serves for the detection of that sequence and determination of level of expression thereof. The techniques to be used for this procedure are described in the art and correspond to the PCR, qPCR and RT-PCR techniques.

### Biological samples

In the present invention, the disclosed method comprises obtaining a biological sample from a subject, wherein said biological sample may be any material containing tissues, cells, nucleic acids, genes, gene fragments, expression products, gene products (e.g., mRNA or proteins) or gene product fragments from a subject to be analyzed. A sample may include, among others, tissue, cells, or biological material of cells or derived from cells of an individual. The sample may be a heterogeneous or homogeneous population of cells or tissues. In certain embodiments, the biological sample is a tissue sample, e.g., a sample obtained from the thyroid or a thyroid nodule of a subject. In particular embodiments of the present invention, a biological sample comprises gene products such as nucleic acids, where mRNA and/or proteins are included.

In the present invention, the subject corresponds to an animal (e.g., a mammal), including but not limited to humans, non-human primates, rodents, dogs, cats, pigs, fish and the like. In particular embodiments, the method is used in biological samples from humans. In particular embodiments, the subject is at risk for or suspected of having a thyroid tumor, wherein being at risk for or suspected of being related to the subject exhibiting one or more symptoms indicative of thyroid cancer (e.g., a noticeable lump or mass) or is being examined for cancer (e.g., during a routine physical exam). The subject can also be a subject suspected of having thyroid carcinoma that can have one or more risk factors. A subject suspected of having thyroid cancer encompasses subjects who have received an initial diagnosis but the stage of the cancer at which the tumor of said subject is found has not been determined or is not known. The term also includes people who have ever had cancer (for example, an individual in remission). Also included are subjects whose biological samples may have been previously analyzed, but the results were not conclusive or indeterminate.

As used herein, the term "subject at risk of thyroid cancer" refers to a subject with one or more risk factors for developing thyroid cancer, in particular aggressive or metastatic thyroid cancer. Risk factors include, but are not limited to, gender, age, genetic predisposition, environmental exposure, previous incidents of cancer, pre-existing non-cancerous diseases, and lifestyle.

It is possible to obtain a biological sample using any method known in the art that can provide a sample suitable for the analytical methods described herein. Methods for obtaining a biological sample from a subject include, but are not limited to, biopsy methods including fine needle aspiration (FNAP), needle aspiration, core needle biopsy, vacuum assisted biopsy, incision biopsy, excision biopsy, or puncture biopsy. In particular embodiments, the methods and kits of the present invention utilize biological samples obtained by FNAP. Methods for obtaining adequate thyroid samples are known in the art and are described in more detail in the ATA International Clinical Guidelines for the Management of Thyroid Nodules (Haugen et al., 2016). Generic methods for obtaining biological samples are also known in the art. In one embodiment, the sample is a fine needle aspiration of a thyroid gland, a thyroid nodule, or a suspected thyroid tumor. In some cases, the fine needle aspiration sampling procedure may be guided by the use of an ultrasound, X-ray, or other imaging device.

In some instances, multiple biological samples, such as multiple thyroid samples, may be obtained for diagnosis by the methods of the present invention, e.g., at the same time or at different times. In some instances, a sample, or samples obtained at the same time or at different times, are stored and/or analyzed by different methods. For example, a sample may be obtained and analyzed by orthological analysis. In some cases, an additional sample may be obtained from a subject at the same time or later, for example, based on the results of an orthology analysis.

The additional sample may be used in a method of the present invention, for example, when the orthological analysis was indeterminate with respect to the presence or absence of cancer. In other embodiments of the methods of the present invention, a single sample and a portion of the sample analyzed may be obtained by orthological analysis, while another portion of the sample is analyzed by the methods of the present invention.

In certain embodiments, a physician may obtain a biological sample from a subject, at locations such as a hospital, medical office, testing center, or laboratory. In certain embodiments, a biological sample may be obtained using a kit, which may contain a means for obtaining a sample as described herein, a means for storing the sample, and instructions for using the kit. In some instances, the kit is provided by a molecular profiling service, which may also perform a diagnostic assay on the biological sample.

In particular embodiments, a biological sample is stored for a time such as seconds, minutes, hours, days, weeks, months, years or more after the sample is obtained and before the sample is analyzed by one or more methods of the invention. In some cases, the sample obtained from a subject is subdivided before the subsequent storage or analysis step, such that different portions of the sample are subject to different subsequent methods or processes, including storage, cytological analysis, suitability testing, nucleic acid extraction, protein extraction, molecular profiling, or combinations thereof. In some instances, one portion of the sample is stored while another portion of said sample is further handled. Such handling may include, but are not limited to: molecular profiling; orthological staining; nucleic acid (e.g., mRNA) extraction, detection, or quantification; protein extraction, quantification; fixation (e.g., formalin-fixed paraffin-embedded samples); and/or examinations. The sample may be fixed before or during storage by any method known in the art, such as the use of glutaraldehyde, formaldehyde or methanol. In other cases, the sample is obtained, stored and subdivided after the storage step for subsequent analysis, such that different parts of the sample are subject to different subsequent methods or processes, including storage, cytological analysis, suitability testing, nucleic acid extraction, polypeptide extraction, molecular profiling, determination of expression of one or more gene products, or a combination thereof. In some cases, samples are obtained and analyzed by, for example, cytological analysis, and the resulting sample material is further analyzed by one or more methods of the present invention comprising determining levels of expression of genetic products described herein, for example, by molecular profiling. In such instances, samples may be stored between the steps of orthology analysis and the steps of determining levels of expression of the gene product, for example, by molecular profiling. In certain embodiments, the samples may be stored frozen, for example, at about any of the following temperatures between about 8°C and about 0°C in a suitable preservation medium during and after sample collection.

The assessment of the biological sample may be performed during or after the obtaining thereof including after the storage process. The assessment of the sample will comprise the determination of its suitability for use in the methods and compositions described in this invention, thereby indicating whether or not the sample is suitable for subsequent analysis. An inadequate sample will be considered if the characteristics of this sample include insufficient cells, insufficient genetic material, insufficient proteins, insufficient DNA or RNA, cells inappropriate for the indicated test, or material inappropriate for the indicated test, age of the sample, way in which the sample was obtained, or way in which the sample was stored or transported. The suitability of the sample may be determined using known methods such as cell staining, measurement of the number of cells or amount of tissue, determination of total proteins and/or nucleic acids, microscopic examinations, temperature and pH assessments. In one embodiment, the adequacy of the sample is determined from the results of performing an analysis experiment of level of gene product. Examples of methods for determining that a suitable number of a specific type of cell is present include PCR, quantitative PCR, RT-PCR, immunohistochemistry, cytological, microscopy, and visual analysis.

Samples may be analyzed by determining the nucleic acid content after extraction of the biological sample using a variety of methods known in the art, such as, for example, ultraviolet absorbance, including, but not limited to, absorbance at 260 nanometers (nm) using a spectrophotometer, among others. In some embodiments, the protein content of the biological sample is determined using various methods known in the art, wherein include but are not limited to ultraviolet absorbance at 280 nm, cellular or protein staining, in some cases protein is extracted from the biological sample prior to measurement thereof. In other embodiments, the samples may be analyzed by protein techniques. In particular embodiments, the samples are processed by any already known method. In particular embodiments, the gene products are selected from nucleic acids, such as DNA and RNA, where mRNA and proteins are included.

### Cytological analysis

Biological samples may be analyzed by cell staining techniques and microscopic observation. Cellular staining may comprise the techniques of EA staining, hematoxylin staining, cytostain, Papanicolaou stain, eosin stain, Nissl stain, toluidine blue, silver stain, azocarmic stain, neutral red or janus green. The nucleic acid content may be determined by staining using ethidium bromide, hematoxylin, NISSL staining or other stains known in the art.

In some embodiments, the cells may be placed on a slide using standard methods known in cytological examinations, furthermore, liquid based cytology (LBC) methods may be used, wherein, biological samples from the subject are transferred to a container or vial containing the liquid orthological solution, which may be any of those known in the art. The sample with the orthological solution may be stored and/or processed for the production of a layer of cells on a glass slide, which in turn may be stained and subsequently observed under a microscope.

In some embodiments, samples may be analyzed by immunohistochemistry assays and thereby observe the presence, location, and distribution of molecules such as proteins, peptides, nucleic acids, or other molecules that may be recognized by specific antibodies, wherein, depending on the antigen to be identified, secondary antibodies may or may not be used for identification.

Cytological examination can determine whether a biological sample is negative or cancer-free; suspicious, ambiguous or suggestive for the presence of cancer; diagnostic (showing the presence of cancer); non-diagnostic or indeterminate (providing inadequate information about the presence or absence of cancer). The results of these diagnoses will determine whether they are benign or malignant, being in some cases diagnoses indicative of the presence of a type of cancer, particularly a type of thyroid cancer.

### KITS

In particular embodiments, the present invention provides diagnostic kits or tests for diagnosing or predicting cancer, such as, for example, thyroid cancer in subjects. The diagnostic tests described herein may be *in vitro* diagnostic tests. Diagnostic tests include, among others, *in vitro* diagnostic tests approved or authorized by the FDA, tests developed in a centralized laboratory, direct-to-consumer tests, which may be used to analyze a biological sample and detect or indicate the presence or absence of metastatic cancer, such as thyroid cancer with lymph node metastasis. In one embodiment, a diagnostic test or kit may be used in a laboratory or other healthcare professional setting. In another embodiment, a consumer may use a home diagnostic test or kit.

The diagnostic tests or kits comprise one or more reagents for the detection of gene products described herein and may comprise other reagents, instruments and systems intended for the *in vitro* diagnostic use of thyroid cancer diagnosis, for the purpose of curing, mitigating, treating or preventing the disease or its sequelae. In one embodiment, the diagnostic kits or tests described herein may be intended for use in the collection, preparation and examination of specimens taken from the human body. As used herein, the term "laboratory test" refers to one or more medical or laboratory procedures that involve analyzing a biological sample obtained from a subject.

The kits and diagnostic tests of the present invention comprise one or more of the reagents for the detection of one or more gene products described herein, such as those expressed by the genes listed in Table 2. In this regard, the reagents for detection may comprise any reagent known to the skilled person for detecting gene products, including, but not limited to, antibodies and oligonucleotides. In certain embodiments, the diagnostic kit or assay may further comprise written instructions on how to perform the assay described herein to determine levels of expression of the gene product using the kit.

In certain embodiments, a diagnostic kit or assay of the present invention comprises two or more, three or more, or four or more reagents, e.g., probes for the detection of a gene product described herein. In particular embodiments, the gene products are proteins or nucleic acids, e.g., mRNA. In certain embodiments, the reagents are antibodies or oligonucleotides, including any of those described herein. In certain embodiments, each reagent is a set of oligonucleotides, e.g., where each set consists of two nucleotides which together are capable of amplifying a target polynucleotide gene product by PCR. In certain embodiments, a diagnostic kit or assay comprises two or more, three or more, or four or more reagents for detecting a gene product, wherein the diagnostic kit or assay comprises two or more, three or more, or four or more sets of primers, each set having the ability to amplify at least a portion of a target gene product. In certain embodiments, said diagnostic kit or assay further comprises two or more, three or more, or four or more separately labeled probes, each probe specifically binding to one of the target gene products or a complement thereof. Accordingly, in certain embodiments, each set of primers is used to amplify a target gene product and then each probe is used to detect amplification of the products and measure levels of expression of each gene product. In particular embodiments, each reagent, e.g., each primer set detects a different gene product. However, it is understood that certain embodiments may include two or more reagents that amplify the same gene product. For example, a diagnostic kit or assay may comprise two reagents, one is a set of amplification primers and the other is a probe, which each specifically binds and/or detects the same gene product. Additionally, a diagnostic kit or assay may comprise multiple combinations of two or more reagents that each specifically binds or detects the same gene product, e.g., wherein each combination specifically binds or detects a different gene product, thereby allowing amplification and detection of multiple gene products, e.g., at the same time.

In particular embodiments, the gene products detected by the reagents of the diagnostic kit or assay are expressed by one or more genes listed in Table 2, wherein at least one of the gene products is expressed by the genes PTEN, BRAF, CD80, CTLA4, VEGFC, miR-16, miR-146b, miR-155, miR-181d, RNU6B, and RET.

In certain embodiments, the diagnostic kit or assay comprises each reagent in a separate container. In other embodiments, each reagent is provided in the same container. In particular embodiments, the reagents are each attached to discrete regions of a solid substrate. Accordingly, in one embodiment, the reagents are oligonucleotides covalently attached to a solid substrate, wherein the array is optionally a microarray. In certain embodiments, the reagents are oligonucleotide sets, e.g., primers, and the oligonucleotide sets comprise DNA.

The kits or diagnostic assays of the present invention may further comprise one or more solutions suitable for binding such reagents to such gene products, and/or one or more solutions or reagents used in performing a method of the present invention for determining an level of expression of the genetic products. For example, in particular embodiments, a diagnostic kit or assay comprises a set of PCR primers, may further comprise one or more additional reagents for performing a PCR assay, such as, for example, a thermostable polymerase, a mixture of deoxynucleotides, and/or a detectably labeled probe. In a particular embodiment, the detectably labeled probe comprises a fluorophore and a cooling moiety, and the probe may emit a detected signal when the probe is cleaved, but not when the probe is intact.

The diagnostic kits or assays of the present invention may further comprise one or more reagents for processing and/or storing a biological sample, e.g., wherein processing the thyroid tissue sample comprises extracting the gene products from the biological sample.

The diagnostic kits or assays of the present invention may further comprise one or more control gene products, such as, for example, positive controls containing a sample of the gene product and/or a negative control that does not contain it, to confirm that the methods performed were successful in specifically identifying and/or measuring the expression and/or presence of gene products.

In certain embodiments, a diagnostic kit or assay comprises data or information, e.g., corresponding to gene levels of expression of the gene products in positive and/or negative control samples or predetermined gene expression cut-off values indicative of the presence or absence of lymph node metastasis in cases of thyroid cancer.

In particular embodiments, one or more reagents for detection of gene products, solution, additional reagents and control gene products are in separate containers.

### DESCRIPTION OF THE DRAWINGS.

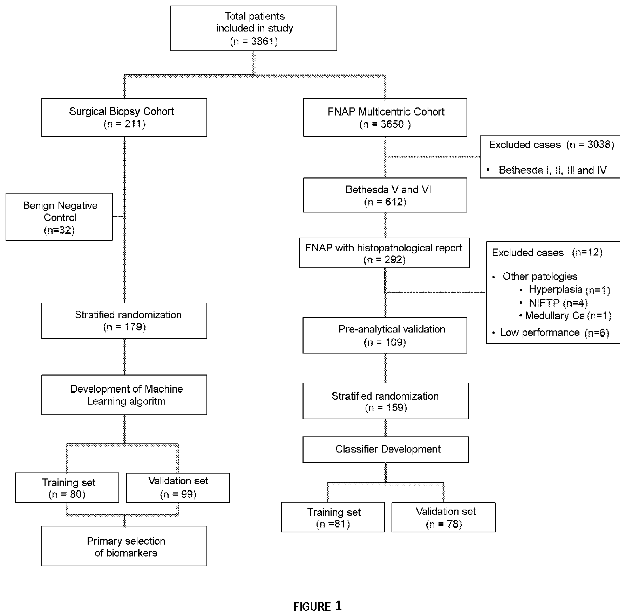

**Figure 1****. Flowchart of the study cohort.** The diagram designed for cohort analysis of thyroid nodule tissue biopsy and FNAP samples is presented in the figure. Samples from each subtype were randomly assigned to the training or validation cohort.

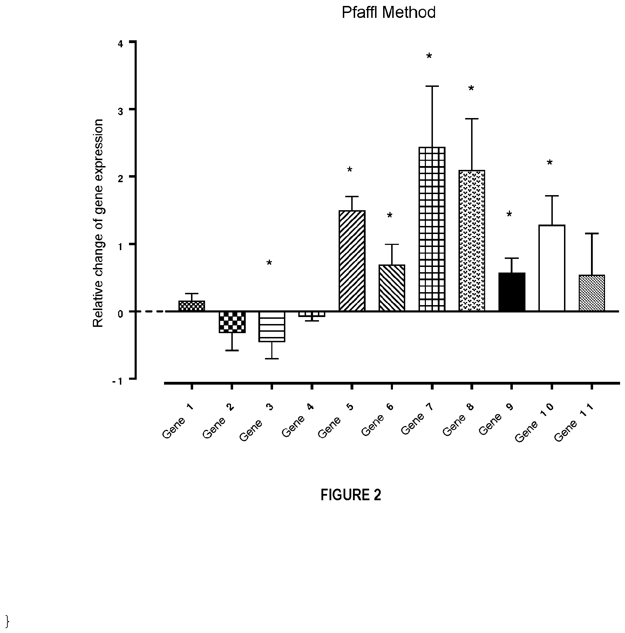

**Figure 2****. Differential expression between FNAP samples with metastatic and non-metastatic papillary thyroid cancer.** Gene expression was determined by quantitative polymerase chain reaction (qPCR) in 62 metastatic and 97 non-metastatic FNAPs. To calculate the gene expression of each sample, the informational gene was normalized by 2 reference genes. The bars represent the gene expression of the metastatic samples with respect to the average of the gene expression of those that do not metastasize (*p < 0.05).

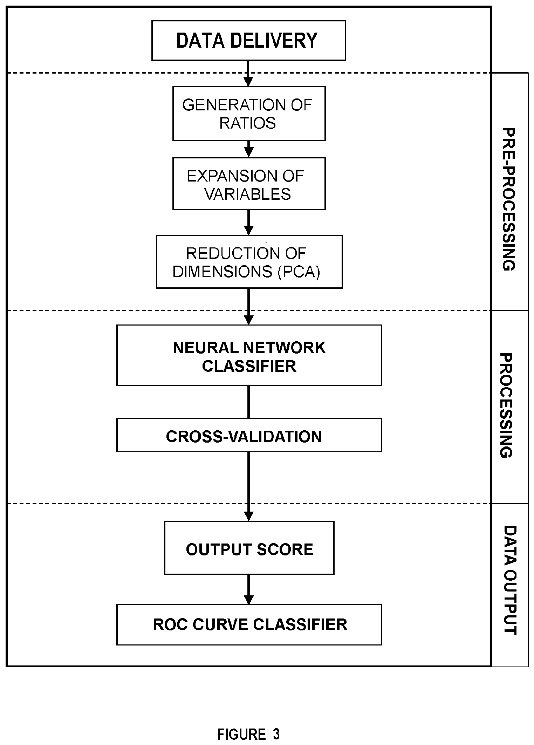

**Figure 3****. Thyroid Metastatic Classifier Model.** The diagram of the gene prediction model is presented in the figure. The analysis of the genes was performed in three consecutive steps. The analysis steps are presented as a) raw data processing using the principal component analysis (PCA) method that simplifies the complexity of the data by maintaining the trends and guidelines, b) data processing using the multi-layer perceptron (MLP) neural network as a strategy for the construction of the classification model, and c)the classification of the data according to score obtained by MLP were integrated for the assessment of the performance of the predictive model. PCA: principal component analysis.

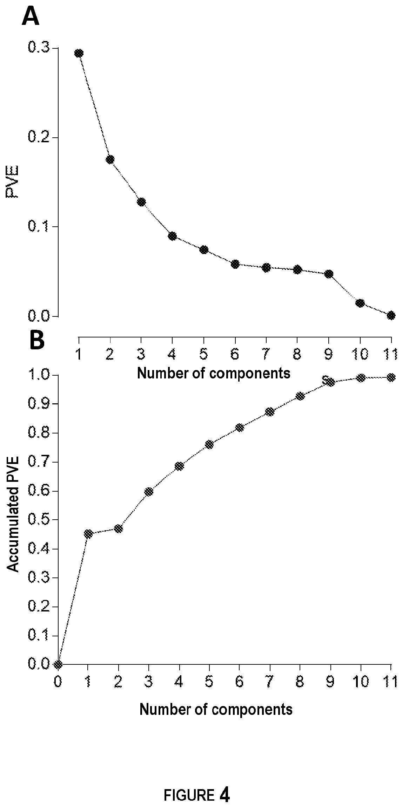

**Figure 4****. Analysis of principal components of the independent variables included in the Thyroid Metastatic Classifier model.** (a) Proportion Explained Variance (PVE) and (b) proportion of cumulative explained variance for each of the principal components. The first 11 principal components obtained are able to explain more than 90% of the total variance of the analyzed data.

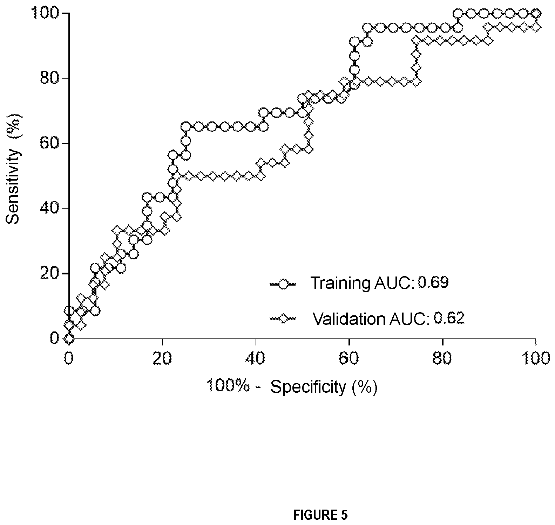

**Figure 5****. ROC curve and area under the curve (AUC) of classification of lymph node metastasis in FNAP by the 5 genes with higher individual AUC values (AUC>0.60) assessed by linear discriminant analysis.** The gene expression of the markers was determined with the qPCR-SYBRGreen technique. The classifier achieved an AUC of 0.69 at the training step and 0.62 at the validation step.

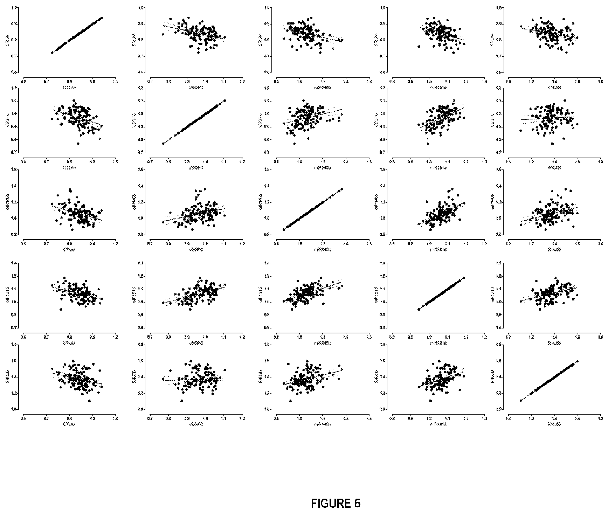

**Figure 6****. Representation of the association between the expression of the 5 genes with higher individual AUC values (AUC >0.60).** Scatter plots showing the correlation between the expression values of genes presenting higher AUC independently. Data analyzed by Spearman correlation coefficient test.

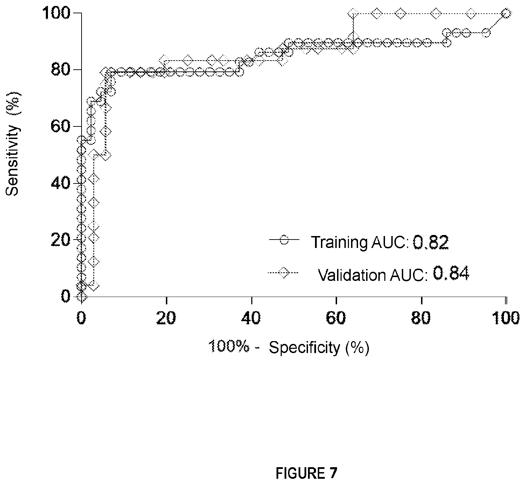

**Figure 7****. ROC curve and area under the curve (AUC) for the Thyroid Metastatic Classifier by qPCR-SYBRGreen technique in FNAP.** A graph showing the area under the curve of the 11 genes used in the predictive thyroid classification model using MLP is presented in the figure. The classifier achieved an AUC of 0.82 at the training step and 0.84 at the validation step.

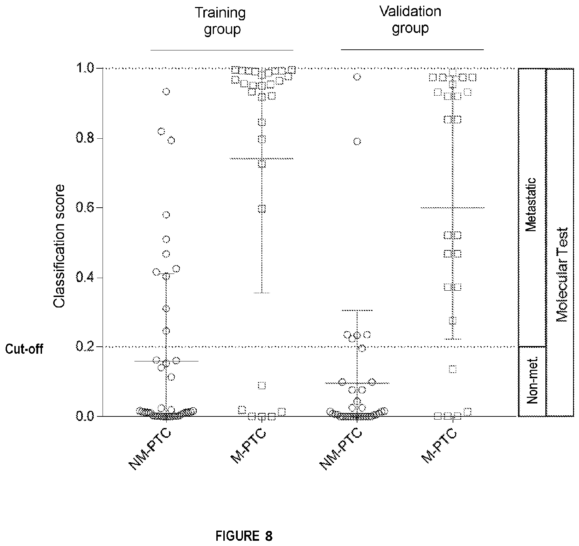

**Figure 8****. The Thyroid Metastatic Classifier effectively identifies metastases by qPCR-SYBRGreen technique in FNAP.** Scatter plot showing the ranking score obtained by the algorithm for each FNAP in the training and validation set, respectively. The classification cut-off between metastasis and non-metastasis was 0.20. The dots show the classification of cases that do not have metastasis (NM-PTC) and squares, those that are metastatic (M-PTC).

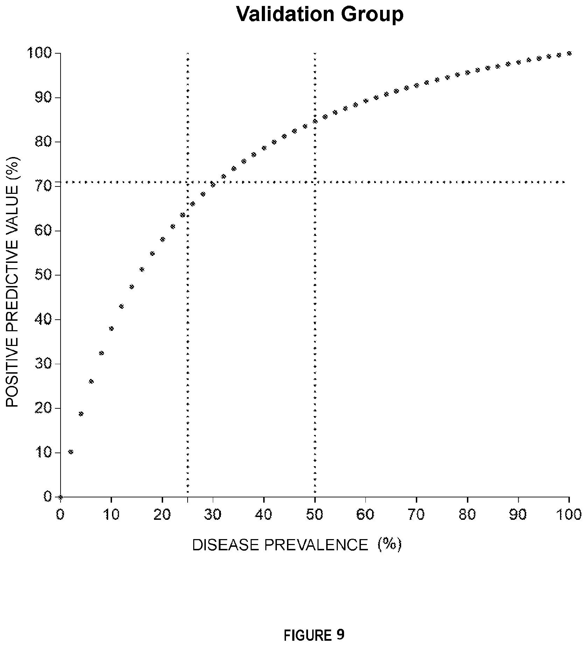

**Figure 9****. Analysis via Bayes' Theorem shows adequate theoretical performance for the Thyroid Metastatic Classifier in the validation step.** By Bayes' theorem, the classifier is expected to reach a VPP of 64-79% and a VPN of 94-82% with a prevalence of lymph node metastasis between 25-40%.

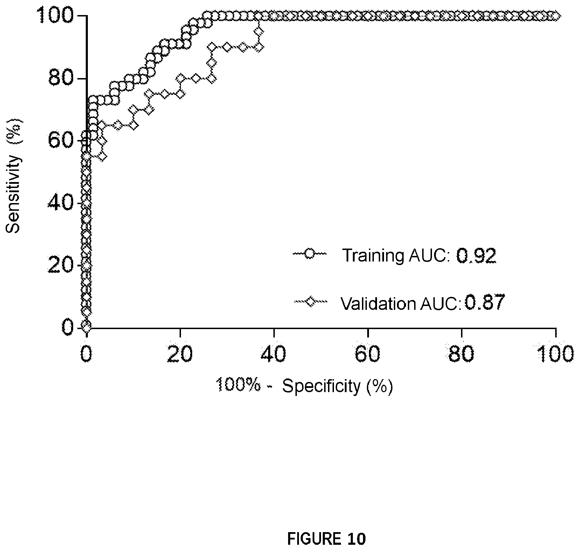

**Figure 10****. ROC curve and area under the curve (AUC) for the Thyroid Metastatic Classifier (11 genes) by qPCR-TaqMan technique in FNAP.** A graph showing the area under the curve of the 11 genes used in the predictive thyroid classification model using MLP is presented in the figure. The classifier achieved an AUC of 0.92 at the training step and 0.87 at the validation step.

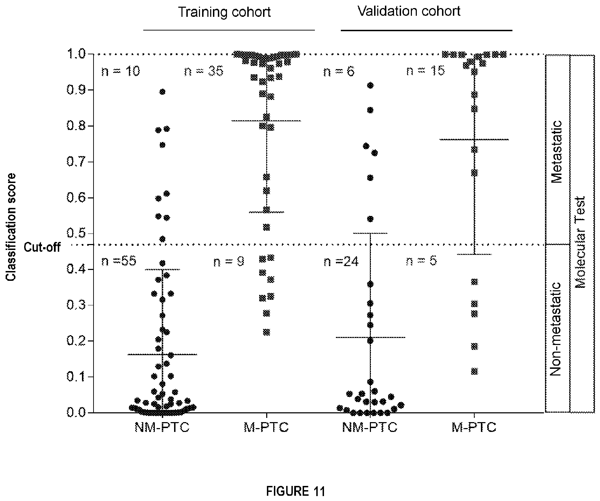

**Figure 11****. The Thyroid Metastatic Classifier effectively identifies metastases by qPCT-TaqMan technique in FNAP.** A scatter plot is presented showing the ranking score obtained by the algorithm for each FNAP. The cut-off of classification for data obtained using the qPCR-TaqMan method between metastases and non-metastasis was 0.47. The dots show the classification of cases that do not have metastasis (NM-PTC) and in squares, those that are metastatic (M-PTC).

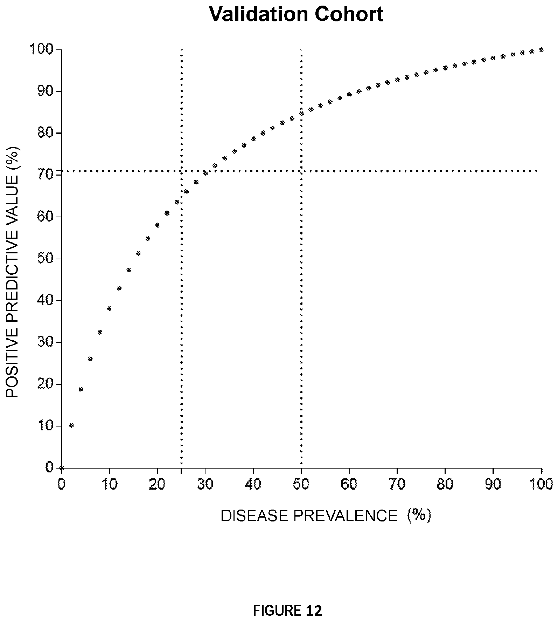

**Figure 12****. Analysis via Bayes' Theorem shows adequate theoretical performance for the Thyroid Metastatic Classifier in the validation step.** By Bayes' theorem, the classifier is expected to reach a VPP of 60-79% and a VPN of 90-79% with a prevalence of lymph node metastasis between 25-40%.

### EXAMPLES OF APPLICATION

### Example 1: Definition of biomarkers for genetic classifier of samples of thyroid nodules in tissue biopsies.

- Collection of thyroid biopsy samples from fresh tissue and cohort of patients Thyroid biopsies of fresh tissue (hereinafter referred to as "tissue biopsies") prospectively between 2014 and 2017 in the clinical hospital of the Pontifical Catholic University of Chile, Santiago, Chile. Tissue biopsies were collected at the operating room of patients undergoing lobectomy or total thyroidectomy and were arranged immediately on RNALater (Ambion).

Samples obtained by Fine Needle Aspiration (FNAP) were collected prospectively using ultrasound guidance between 2016 and 2018 in six centers academics, were stabilized using RNAprotect cell reagent (Qiagen) and stored at -80°C until RNA extraction. Patients signed a consent informed prior approval by each institution's Ethics Committee. Surgical histopathology report was used as the gold standard to compare cytologies both for tissue biopsies and FNAPs in the Bethesda V and VI categories.

A flowchart of the study design is presented in figure 1, where samples from each subtype were randomized to the training cohort or the validation cohort.

The minimum sample size for each set was calculated based on World Health Organization recommendations for clinical studies with 80% potency and 95% accuracy for testing. In addition, an operator blind to the molecular diagnosis compared the score of the classifier with the respective surgical pathology report for each patient.

After a follow-up of at least 4 years of a metacentric cohort of 3650 FNAP samples, the surgical histopathological report (gold standard) was obtained in 292/612 cases of cancer (Bethesda V/VI). Next, the samples were stratified according to the cytology report and the aggressiveness characteristic to be studied as a dependent variable (metastatic/non-metastatic) and randomized. The clinical-histopathological characterization of the FNAP samples is presented in Table 1.

| | | | **PAAF (n=159)** | |
|---|---|---|---|---|
| **Sex (n, %)** | | | | |
| | Male | 33 | | 21 % |
| | Female | 125 | | 79 % |
| **Age (years)** | | | 42 ± 13 | |
| **Size** | | | | |
| | <2 | 135 | | 85% |
| | 2-4 | 24 | | 15% |
| | >4 | 0 | | 0% |
| **Histology** | | | | |
| | CPT-vU | 89 | | 56 % |
| | CPT-vF | 25 | | 16% |
| | CPT-vHC | 5 | | 3% |
| | CPT-vTC | 27 | | 17 % |
| | CPT-vH | 6 | | 4 % |
| | CPT-vWL | 5 | | 3 % |
| | CPT-vMC | 2 | | 1 % |
| **Multifocus** | | | | |
| | No | 87 | | 55% |
| | Yes | 54 | | 34 |
| **Extrathyroidal extension** | | | | |
| | No | 105 | | 66 % |
| | Yes | 54 | | 34 % |
| **Lymph Node Metastasis** | | | | |
| | No | 95 | | 60% |
| | Central | 45 | | 28 % |
| | Lateral | 19 | | 12 % |

| | | | | |
|---|---|---|---|---|
| PAAF: Fine needle aspiration puncture. PTC: papillary thyroid carcinoma, vU: usual variant, vF: follicular variant, vHC Hürthle Cell variant, vTCV: Tall Cell variant | | | | |

### - RNA extraction and cDNA synthesis

Total RNA from tissue biopsies and FNAP was extracted with the RNeasy Plus-Mini kit (Qiagen). The total RNA concentration was determined using the Qubit^{®} RNA HS Assay Kit and the Qubit^{®} 2.0 Fluorometer (Invitrogen). Reverse transcription (RT) reactions to assess mRNA expression was performed in a final volume of 20 µL using 150 ng RNA total tissue biopsies or 50 ng FNAP with the reverse transcription system Improm II^{™} (Promega), following the manufacturer's instructions.

RT reactions to assess miRNAs were performed according to the miQPCR method published in Benes et al., 2015. Briefly, a denaturation mixture was prepared containing 10 ng total RNA, 0.4 µM miLINKER adapter and 30% PEG 8000 (New England Biolabs), and incubated for 3 minutes at 75°C. Next, the adapter miLINKER was ligated to template miRNAs for 30 minutes at 25°C in the presence of T4 buffer 1X (New England Biolabs), 5 µM MgCl2, 0.1 µL RNase inhibitor 40 U/µL (Promega) and 200 T4 RNA units Ligase 2, truncated K227Q (New England Biolabs).

cDNA synthesis was performed in three steps: first, the mixture was incubated with 0.25 mM dNTPs and 0.125 µM mQ-RT universal primer for 2 minutes at 85°C; then added 1X RT buffer (Invitrogen), 5 mM DTT (Invitrogen) and water to complete one volume of 19.7 µL were added to the mixture and incubated for 3 minutes at 45°C. Finally, 0.3 µL of SuperScript II (200 U/µL; Invitrogen) was added to the mixture, incubated for 30 minutes at 45°C, 3 minutes at 85°C and maintained at 10°C.

### - Selection of biomarkers

For the pre-selection of the biomarkers to be assessed, reported genes showing differential expression and/or biological importance in the aggressiveness/inflammation/epithelial-mesenchymal transition of thyroid carcinogenesis were identified through a PubMed search (www.ncbi.nlm.nih.gov/pubmed/). Genes BRAF (gene 1), CD80 (gene 2), CTLA4 (gene 3), PTEN (gene 4), VEGFC (gene 5), miR-16 (gene 6), miR-146b (gene 7), miR-155 (gene 8), miR-181d (gene 9), RNU6B (gene 10), RET (gene 11) are included. These genes are described in Table 2, wherein the sequence identifier is further indicated for the present invention.

### -qPCR

To assess mRNA expression in tissue biopsy samples, the reactions of qPCRs were performed in a final volume of 20 µL of reaction mixture containing 2 µL of cDNA, 10 µL of 2X Brilliant II SYBR Green qPCR Master Mix (Agilent Technologies), 250 nM of each primer and nuclease free water.

The conditions for the amplification reaction were: 10 min at 95°C, followed by 40 cycles of 20 sec. at 95°C, 20 sec. at 60°C and 20s at 72°C. The melting curve analysis was performed by increasing the temperature 1°C/s in the range from 72°C to 95°C.

To assess miRNA expression, qPCR reactions were performed in 20 µL volumes with 0.1 ng of synthesized cDNA (100 µg/reaction) in the presence of a 0.25 µM miQPCR-specific universal reverse primer, 1X Brilliant II SYBR Green Mastermix (Agilent Technologies) and nuclease-free water to complete 20 µL of reaction. The reaction of 10 qPCRs was performed in the Rotor-Gene Thermocycler (Qiagen) under the following reaction conditions: 95°C for 10 minutes, 50 cycles of 95°C for 10s, 60°C for 35 sec., and a final step for the melting curve from 60°C to 95°C, increasing by one degree per second (Table 3).

**Table 2: Description of the 11 genes of the Thyroid Metastatic Classifier selected by qPCR-SYBRGreen technique.**

| **Genes** | **Full name** | **Other names** | **Access number (Ref Seq)** | |
|---|---|---|---|---|
| | | | **Polynucleotide** | **Polypeptide** |
| BRAF (Gene 1) | B-raf serine Proto-oncogene/Threonine kinase | NS7, B-raf.BRAF1,RRAFB1,B-RAF1 | NM_004333.6 (SEQ ID NO:1) | NP_004324.2 (SEQ ID NO:2) |
| CD80 (Gene 2) | CD28 Antigen Ligand 1 | Antigen B7-1, BB1, B7-1, B7.1, LAB7, CD28LG, CD28LG1 | NM_005191.3 (SEQ ID NO:3) | NP_005182.1 (SEQ ID NO:4) |
| CTLA4 (Gene 3) | Antigen 4 cytotoxic T-lymphocyte-associated | CD, GSE, GRD4, APS5, CD152, IDDM12, CELIAC3 | NM_005214.5 (SEQ ID NO:5) | NP_005205.2 (SEQ ID NO:6) |
| PTEN (Gene 4) | Alkaline phosphatase homologue | BZS, DEC, CWS1, GLM2, MHAM, TAP1, MMAC1, PTEN1, 10q23del, PTENbeta | NM_000314.8 (SEQ ID NO:7) | NP_000305.3 (SEQ ID NO:8) |
| VEGFC (Gene 5) | Factor of vascular endothelial growth C | VRP, Flt4, LMPH1D, LMPHM4 | NM_005429.5 (SEQ ID NO:9) | NP_005420.1 (SEQ ID NQ:10) |
| miR-16 (Gene 6) | hsa-miR-16-5p | - | MIMAT0000069 (SEQ ID NO:11) | - |
| miR- 146b (Gen 7) | hsa-miR-146-5p | - | MIMAT0002809 (SEQ ID NO:12) | - |
| miR-155 (Gene 8) | hsa-miR-155-5p | - | MIMAT0000646 (SEQ ID NO:13) | - |
| miR- 181d (Gene 9) | hsa-miR-181d-5p | - | MIMAT0002821 (SEQ ID NO:14) | - |
| RNU6B (Gene10) | U6, small nuclear RNA 6 | U6-6, RNU6-B, RNU6- 6 | NR_002752.2 (SEQ ID NO:15) | - |
| RET (Gene 11) | Ret proto-oncogene | cRET | NM_020975 (SEQ ID NO:16) | NP_066124.1 (SEQ ID NO:17) |

To avoid genomic amplification, primers used for analysis of tissue biopsies and FNAP were designed in different exons using Primer-BLAST software (www.ncbi.nlm.nih.gov/tools/primer-blast/).

In FNAP samples, gene expression of biomarkers was assessed by qPCR in multiplex configuration using TaqMan probe assays, including one target gene and two reference genes. Reactions were standardized following the manufacturer's recommendations (https://tools.thermofisher.com/content/sfs/manuals/taqman_optimization_man.pdf). QPCR reactions to assess mRNA were performed by adding 5 µl of 1:12.5 RT reaction dilution in a 20 µl final volume containing 10 µl of 2X TaqMan ^{™} multiplex master mix with Mustang Purple (Applied Biosystems), sequence-specific TaqMan ^{™} assays (Applied Biosystems), and nuclease-free water. RT reactions to assess miRNAs were performed using the miRNA Advanced TaqMan^{™} cDNA Synthesis Kit (Applied Biosystems) and miRNA expression was quantified by adding 5 µL of RT 1:20 reaction dilution into a 20 µL final volume containing 10 µL of TaqMan^{™} Multiplex 2X Master Mix with Mustang Purple (Applied Biosystems), sequence-specific Advanced TaqMan^{™} miRNA assays (Applied Biosystems), and nuclease-free water. The conditions for the amplification were: 20 seconds at 95°C, followed by 40 cycles of 3 seconds at 95°C and 30 seconds at 60°C.

All qPCR reactions were performed in the Rotor-Gene Q thermocycler (Qiagen). Reactions with a cycle threshold > 35 and/or poor melting curves were not considered.

**Table 3. Primer sequences for SYBRGreen testing and TaqMan^{™} probe assays for the target genes.**

| **Genes** | **Primer Sequence** | **qPCR product size (bp)** | **Probe test ID** |
|---|---|---|---|
| BRAF (gene 1) | S: 5'TCATGAAGACCTCACAGTAAAAATA3' A: 5'TGGTGCCATCCACAAAATGG 3' | 115 | Hs00269944_m1 |
| CD80 (gene 2) | S: 5'AACAGTGTCCGCAGAAGCAA 3' A: 5'AGGGCAAGGTGGGGTAATCT 3' | 112 | Hs01045161_m1 |
| CTLA4 (gene 3) | S: 5'AACACCGCTCCCATAAAGCC 3' A: 5'GACCTCAGTGGCTTTGCCT 3' | 227 | Hs00175480_m1 |
| PTEN (gene 4) | S: 5'CTCAGCCGTTACCTGTGTGT 3' A: 5'AGGTTTCCTCTGGTCCTGGT 3' | 129 | Hs02621230_s1 |
| VEGFC (gene 5) | S: 5'CCTCGGATGCTGGAGATGAC 3' A: 5'CACATTGGCTGGGGAAGAGT 3' | 192 | Hs01099203_m1 |
| miR-16 (gene 6) | S: 5'TAGCAGCACGTAAATATTGGCG 3' | 22 | 477860_mir |
| miR-146b (gene 7) | S: 5'TGAGAACTGAATTCCATAGGCT 3' | 23 | 483144_mir |
| miR-155 (gene 8) | S: 5'TTAATGCTAATCGTGATAGGGGT 3' | 24 | 483064_mir |
| miR-181d (gene 9) | S: 5'AACATTCATTGTTGTCGGTGGGT 3' | 23 | 479517_mir |
| RNU6B(gene 10) | S: 5'GCAAGGATGACACGCAAATT 3' | 42 | 001093 |
| RET (gene 11) | S: 5'AATTTGGAAAAGTGGTCAAGGC3' | 83 | Hs01120030_m1 |

| | | | |
|---|---|---|---|
| *bp: Base Pairs | | | |

Table 3 presents the primer sequences for use in SYBRGreen and identification of the probe assays for use in TaqMan of the 11 selected genes. S: sense splitter; A: antisense splitter.

A graph (Figure 2) is provided showing the differential expression between samples of fine needle aspiration biopsy (FNAP) metastatic (62) and non-metastatic (97) of 10 genes as determined by quantitative real-time PCR and analyzed by the model of relative quantification of Pfaffl. To calculate the gene expression of each sample, the target was normalized with 2 reference genes. The zero value corresponds to the expression gene of thyroid tumors without metastasis and the bars correspond to the variation of the gene expression in metastatic cancer samples (relative times of change). The results of gene expression of the 11 genes analyzed by Pfaffl's indicate that 6 genes (VEGFC, miR-16, miR-146, miR-155, miR-181d, RNU6B) were significantly overexpressed (p<0.05), while 1 gene (CTLA4) decreased its expression significantly compared to non-metastatic CPT (p<0.05) (Figure 2).

### Example 2: Design and development of a model of thyroid metastasis classifier for diagnostic/prognostic test

In order to identify a panel of differentially expressed genes, a machine learning model according to the network analysis algorithm neural networks (NN) (Keras package, a statistical library and a library of networks open-source neural networks; https: //cran.r-project.org/web/packages/keras/index.html), using R v3.3.1 software. (Figure 3)

The prediction was made from 81 FNAP samples for the training step and 78 FNAP samples for the validation step. Previously, to optimize the performance of the classifier, input or predictor variables (gene expression) were assessed by a principal component analysis (PCA; kernlab package; https://cran.r-project.org/web/packages/kernlab/index.html). This technique reduces the dimensionality of data, finding the causes of variability and defining patterns predominant in a data set. In particular, in Figure 4, a graph is presented showing the first 11 major components explaining 99.3% of the variance between the variables.

Then a second data processing was performed in which an algorithm was used Multilayer Perceptron (MLP) Neural Network ") and with this the predictive model was built (figure 3). The construction of the final prediction model was performed following the method of training and cross validation of the sensors with different combinations of hyperparameters. The hyperparameters considered were epoch, learning rates, dropouts, rho and epsilon. Of all the randomized data, we used 50% for the training step and the 50% for cross-validation using the SYBRGreen gene expression quantification technique. Using the TaqMan gene expression quantification technique, we used 70% of the data for the training step and 30% for the cross-validation step. The best network with an accuracy > 0.80 was chosen for the training cohort and of cross-validation.

To validate the diagnostic efficacy of the genetic signature in classifying cases with metastatic and non-metastatic thyroid cancer, the receiver operating characteristics (ROC) curve with the output scores obtained from the analysis carried out with the MLP algorithm, using the ROCR R package, http://rocr.bioinf.mpi-sb.mpg.de/. This method was used for both the data obtained by the SYBRGreen (qPCR-SYBRGreen) as for those obtained by the TaqMan technique (qPCR-TaqMan).

Finally, to obtain the prediction method, the scores of the output data obtained from analysis with the MLP algorithm were classified for assessment of diagnostic efficacy of Thyroid Metastatic Classifier (CMT).

In the first instance, the potential classifying power of gene expression was compared of markers individually in FNAP samples with thyroid cancer metastatic and non-metastatic, by elaboration of ROC curves. For each individual gene, ROC curves were made from the CT values. In Table 4, presents the area under the curve (AUC) for each gene as result of individual diagnostic efficacy. The diagnostic performance of each gene shown in this table and it is observed that none of the genes assessed individually reached the minimum performance required to classify metastatic thyroid cancer from non-metastatic cases. However, VEGFC showed the largest relative change in the gene expression and best diagnostic performance (AUC> 0.70).

**Table 4. Individual diagnostic performance of the 11 genes selected by the qPCR-SYBRGreen technique.**

| **Gene No.** | **Gene Name:** | **AUC value** | **Sensitivity** | **Specificity** |
|---|---|---|---|---|
| 1 | BRAF | 0,532 | 42 % | 63 % |
| 2 | CD80 | 0,575 | 50 % | 59 % |
| 3 | CTLA4 | 0,612 | 53 % | 77 % |
| 4 | PTEN | 0,503 | 42 % | 60 % |
| 5 | VEGFC | 0,709 | 50 % | 80 % |
| 6 | miR-16 | 0,534 | 42 % | 59 % |
| 7 | miR-146b | 0,629 | 42 % | 73 % |
| 8 | miR-155 | 0,514 | 42 % | 69 % |
| 9 | miR-181d | 0,612 | 42 % | 73 % |
| 10 | RNU6B | 0,649 | 41 % | 76 % |
| 11 | RET | 0,505 | 42 % | 61 % |

| | | | | |
|---|---|---|---|---|
| *AUC: area under the curve. | | | | |

To verify that the diagnostic potential is not by mere additive combination of expression factors of the markers or by a linear relationship between the input or predictors, the diagnostic efficacy of the expression of the 5 genes that presented individual AUC values greater than 0.60, by linear discriminant analysis (LDA). Figure 5 shows that the AUC obtained for the training cohort is 0.69 and for that of validation, of 0.62.

Table 5 presents the data that represent the diagnostic efficacy that determined from the classification results obtained by ALD from the expression of the 5 genes with the highest single AUC. In the training cohort, the diagnostic efficacy of the classifier reached an AUC of 0.69 (CI 0.56 - 0.84), a sensitivity of 65% (CI 43 - 84), and a specificity of 69% (CI 52 - 84). The VPP and VPN were from 58% (CI 41 - 74%) and 76% (CI 60 - 87%) respectively. In the validation cohort statistically, an AUC of 0.63 (CI 0.48 - 0.77), a sensitivity of 50% (CI 70 - 96%) was obtained, and a specificity of 62% (CI 45 - 77%). VPP and VPN were 47% (CI 30 - 65%) and 65% (CI 49 - 79%), respectively.

**Table 5. Statistical efficiency of classification of the 5 genes with higher individual AUC values (AUC >0.60) assessed by Linear Discriminant Analysis.**

| | **Training Cohort in FNAP** | | **Statistical Validation Cohort in FNAP** | |
|---|---|---|---|---|
| **Statistical Parameter** | Value | 95%, IC | Value | 95%, IC |
| **Cases** | 81 | | 78 | |
| **AUC** | 0.69 | (0,56-0,84) | 0.63 | (0,48-0,77) |
| **Prevalence of disease** | 39 % | (28-52) | 40 % | (29-51) |
| **Sensitivity** | 65 % | (43-84) | 50 % | (29-71) |
| **Specificity** | 69 % | (52-84) | 62 % | (45-77) |
| **Predictive value positive** | 58 % | (41-74) | 47 % | (30-65) |
| **Predictive value negative** | 76 % | (60-87) | 65 % | (49-79) |
| **Positive likelihood ratio** | 2 | (1-3) | 1 | (0,8-2) |
| **Post Probability positive test** | 58 % | (40-74) | 45 % | (29-63) |
| **Negative likelihood ratio** | 0.49 | (0,30-0,81) | 0.82 | (0,56-1,18) |
| **Post Probability negative test** | 24 % | (13-40) | 35 % | (22-50) |
| **Accuracy** | 70 % | (67-89) | 68 % | (55-78) |

| | | | | |
|---|---|---|---|---|
| AUC: Area under the curve; FNAP: fine needle aspiration puncture; Cl: confidence interval | | | | |

Altogether, these data show poor diagnostic and predictive capacity for lymph node metastasis in FNAP samples from papillary thyroid cancer patients by combining and adding gene expression factors in a linear fashion. This is explained because linear regression analysis methods, such as ALD, are sensitive to collinearity phenomena that can occur between input or predictor variables, that is, a predictor variable may be largely predicted from another predictor variable.

This phenomenon may be seen in the data provided by Table 6 and Figure 6, where the gene expression of the markers CTLA4, VEGFC, miR-146b, miR-188d and RNU6B (predictor variables of lymph node metastasis) present a significant linear correlation between them.

**Table 6. Association between the expression of the 5 genes with higher individual AUC values (AUC >0.60). Correlation analysis between the expression values of the genes that presented the highest AUC independently. Data analyzed by Spearman correlation coefficient test.**

| **Spearman Rho** | | **CTLA4** (Gene 3) | **VEGFC** (Gene 5) | **miR- 146b** (Gene 7) | **miR-181d** (Gene 9) | **RNU6B** (Gene 10) |
|---|---|---|---|---|---|---|
| **CTLA4** (Gene 3) | Correlation coefficient | 1,000 | -0,431** | -0,362** | -0,353** | -0,295* |
| | significance (bilateral) | · | 0,001 | 0,005 | 0,006 | 0,024 |
| **VEGFC** (Gene 5) | Correlation coefficient | -0,431** | 1,000 | 0,380** | 0,504** | 0,170 |
| | significance (bilateral) | 0,001 | · | 0,003 | 0,000 | 0,198 |
| **miR-146b** (Gene 7) | Correlation coefficient | -0,362** | 0,380** | 1,000 | 0,555** | 0,431** |
| | significance (bilateral) | 0,005 | 0,003 | · | 0,000 | 0,001 |
| **miR-181d** (Gene 9) | Correlation coefficient | -0,353** | 0,504** | 0,555** | 1,000 | 0,438** |
| | significance (bilateral) | 0,006 | 0,000 | 0,000 | · | 0,001 |
| **RNU6B** (Gene 10) | Correlation coefficient | -0,295* | 0,170 | 0,431** | 0,438** | 1,000 |
| | significance (bilateral) | 0,024 | 0,198 | 0,001 | 0,001 | |

Figure 7 presents the AUC where the classifier developed in qPCR-SYBRGreen used 11 genes that were integrated by the neural network. In this figure it is possible to observe that the classifier in the training cohort reaches an AUC of 0.82, while in the validation cohort it reaches an AUC of 0.84.

Table 7 presents the data obtained from the samples that were assessed by the qPCR-SYBRGreen method. In the training cohort, the diagnostic efficacy of the classifier reached an AUC of 0.82 (CI 0.73 - 0.92), a sensitivity of 79% (CI 61 - 92), and a specificity of 76% (CI 61 - 88). The VPP and VPN were from 70% (CI 56 - 80%) and 85% (CI 73 - 92%) respectively. In the statistical validation cohort, the classifier reproduced its performance, showing an AUC of 0.84 (CI 0.76 - 0.95), a sensitivity of 72% (CI 70 - 96%), and a specificity of 87% (Cl 61-88%). VPP and VPN were 79% (Cl 59-90%) and 82% (Cl 69-90%), respectively.

**Table 7. Statistical efficacy of the Thyroid Metastatic Classifier developed with the qPCR-SYBRGreen technique.**

| **Statistical Parameter** | **Training Cohort in FNAP** | | **Statistical Validation Cohort in FNAP** | |
|---|---|---|---|---|
| | Value | 95%, IC | Value | 95%, IC |
| Cases | 81 | | 78 | |
| AUC | 0,82 | (0,73-0,92) | 0,84 | (0,76-0,95) |
| Prevalence of disease | 39 % | (29-52) | 40 % | (28-53) |
| Sensitivity | 79% | (61-92) | 72 % | (70-96) |
| Specificity | 76 % | (61-88) | 87 % | (61-88) |
| Positive predictive value | 70 % | (56-80) | 79 | (50-90) |
| Negative predictive value | 85 % | (73-92) | 82 % | (69-90) |
| Reason for positive likelihood | 3 | (2-5) | 6 | (2-12) |
| Positive Post-test Probability | 68 % | (50-81) | 78% | (58-90) |
| Negative likelihood ratio | 0,28 | (0,14-0,56) | 0,33 | (0,19-0,58) |
| Negative post-test probability | 16 % | (7-30) | 18 % | (9-32) |
| Accuracy | 78% | (66-87) | 81 % | (67-90) |

| | | | | |
|---|---|---|---|---|
| AUC: Area under the curve, FNAP: fine needle aspiration puncture, Cl: confidence interval. | | | | |

To observe the diagnostic efficacy of the prediction classifier of papillary thyroid cancer in FNAP samples developed in qPCR-SYBRGreen, the score obtained at the training step and at the validation step was plotted for each of the samples. The cut-off for classifying samples as metastatic (M-PTC) or non-metastatic (NM-PTC) was 0.20. These data may be observed in Figure 8.

The positive and negative predictive values of the Papillary Thyroid Cancer Prediction Classifier for the samples assessed by the qPCR-SYBRGreen technique were estimated according to the Bayes Theorem (Figure 9). The classifier is expected to reach a positive predictive value of 60-79% and a negative predictive value of 94-82% in a range of prevalence of lymph node metastasis of 25-40%.

Figure 10 shows the area under the curve where the classifier developed in qPCR-TaqMan used 11 genes that were developed by the neural network. In this figure it is possible to observe that the classifier in the training step reaches an AUC of 0.92, while in the validation step it reaches an AUC of 0.87.

In Table 8, the data obtained from the Thyroid Metastatic Classifier are presented in FNAP samples that were assessed by the qPCR-TaqMan method. In the training cohort, the diagnostic efficacy of the classifier reached an AUC of 0.92 (Cl 0.91-0.93), a sensitivity of 80% (Cl 63-92), and a specificity of 84% (Cl 70-92). VPP 5 and VPN were 76% (CI 58 - 88%) and 87% (CI 75 - 86%), respectively. In the validation set, the classifier reproduced its performance, showing an AUC of 0.87 (CI 0.85 - 0.91), a sensitivity of 73% (CI 69 - 90%), and a specificity of 81% (Cl 78-93%). The VPP and VPN were from 71% (Cl 52-76%) and 80% (Cl 55-93%) respectively.

**Table 8. Statistical efficacy of the Thyroid Metastatic Classifier developed with the qPCR-TaqMan technique.**

| **Statistical Parameter** | **Training Cohort in FNAP** | | **Statistical Validation Cohort in FNAP** | |
|---|---|---|---|---|
| | Value | 95%, IC | Value | 95%, IC |
| Cases | 109 | | 50 | |
| AUC | 0.92 | (0,91-0,93) | 0,87 | (0,85-0,91) |
| Prevalence of disease | 39 % | (29-51) | 40 % | (25-50) |
| Sensitivity | 80 % | (63-92) | 73 % | (69-90) |
| Specificity | 84 % | (70-92) | 81 % | (78-93) |
| Positive predictive value | 76 % | (58-88) | 71 % | (52-76) |
| Negative predictive value | 87% | (73-95) | 80 % | (55-93) |
| Reason for positive likelihood | 8 | (3-8) | 3 | (2-8) |
| Positive Post-test Probability | 75 % | (60-86) | 71 % | (47-87) |
| Reason for negative likelihood | 0,25 | (0,13-0,45) | 0,31 | (0,14-0,67) |
| Negative post-test probability | 14% | (7-25) | 17 % | (6-36) |
| Accuracy | 77 % | (75-86) | 76 % | (74-85) |

| | | | | |
|---|---|---|---|---|
| AUC: Area under the curve, FNAP: fine needle aspiration puncture, Cl: confidence interval. | | | | |

To observe the diagnostic efficacy of the prediction classifier of papillary thyroid cancer in FNAP samples developed in qPCR-TaqMan, the score obtained at the training and validation step was plotted for each of the samples. The cut-off for classifying samples as metastatic (M-PTC) or non-metastatic (NM-PTC) was 0.47. These data may be observed in Figure 11.

The positive and negative predictive values of the Thyroid Metastatic Classifier for the FNAP samples assessed using the qPCR-TaqMan technique were estimated according to the Bayes' theorem (Figure 12). The classifier is expected to reach a positive predictive value of 60-79% and a negative predictive value of 90-79% in a range of prevalence of lymph node metastasis of 25-40%.

The results of the criteria that determine the diagnostic efficacy obtained by the qPCR-SYBRGreen and qPCR-TaqMan techniques are comparable and even show greater classification power when using the qPCR-TaqMan technique. These results indicate that the prediction classifier for papillary thyroid cancer developed in qPCR- format TaqMan in multiplex configuration is robust and predicts with high diagnostic efficiency standards the lymph node metastasis in FNAP samples pre-surgically.

### Statistical analysis

For tissue biopsies and FNAP, gene expression was analyzed by the Pfaffl method. Sensitivity, specificity, and area under the curve (AUC) were estimated by receiver operating characteristics (ROC). Positive and negative predictive values were estimated by Bayes' theorem. The association analyses among the input or predictor variables were performed with Spearman's correlation coefficient test. Multiple comparison tests were performed using Tukey's rank test. Significant differential expression was estimated by testing Wilcoxon sign ranges. The p-values of two tails of less than 0.05 were considered to indicate statistical significance. The collected data was organized in Microsoft Office Excel 365, the graphics were made with GraphPad statistical software v7.0 (GraphPad Software, Inc.) and all Statistical analysis was performed with SPSS software v23.0 (IBM).

### REFERENCES

Benes V, Collier P, Kordes C, Stolte J, Rausch T, Muckentaler MU, Háussinger D, Castoldi M. Identification of cytokine-induced modulation of microRNA expression and secretion as measured by a novel microRNA specific qPCR assay. Sci Rep. 2015. 25;5:11590.
Faquin WC, Bongiovanni M, Sadow PM. 2011. Update in thyroidfine needleaspiration. Endocr Patho 122:178-183
González H, Martinez J, Vargas-Salas S, Solar A, Veliz L, Cruz F, Arias T, Loyola S, Horvath E, Tala H, Traipe E, Meneses M, Marin L, Wohllk N, Diaz R, Veliz J, Pineda P, Arrollo P, Mena N, Bracamonte M, Miranda G, Bruce E, Urra S. 2017. A 10-Gene Classifier for Indeterminate Thyroid Nodules: Development and Multicenter Accuracy Study. Thyroid. Volume 27, Number 8, 2017
Haugen BR, Alexander EK, Bible KC, Doherty GM, Mandel SJ, Nikiforov YE, et al. 2015 American Thyroid Association Management Guidelines for Adult Patients with Thyroid Nodules and Differentiated Thyroid Cáncer: The American Thyroid Association Guidelines Task Forcé on Thyroid Nodules and Differentiated Thyroid Cáncer. Thyroid. 2016;26:1-133
Jacques C, Guillotin D, Fontaine JF, Franc B, Mirebeau-Prunier D, Fleury A, Malthiery Y, Savagner F.. (2013). DNA Microarray and miRNA Analyses Reinforce the Classification of Follicular Thyroid Tumors. The Journal of Clinical Endocrinology & Metabolism, Volume 98, Issue 5, 1 May 2013, Pages E981-E989.
Jia Liu, Dongmei Zheng, Qiang Li, Xulei Tang, Zuojie Luo, Zhongshang Yuan, Ling Gao y Jiajun Zhao. (2018). A predictive model of thyroid malignancy using clinical, biochemical and sonographic parameters for patients in a multi-center setting. BMC Endocr Disord. 2018; 18: 17.
National Cáncer Institute. Thyroid cancer treatment (PDQ). http://cancer.gov/cancertopics/pdq/treatment/thyroid/HealthProfessional.
Lundgren Cl Hall P, Dickman PW, Zedenius J. (2006). Clinically significant prognostic factors for differentiated thyroid carcinoma. Cáncer. 2006;106:524-531.
Sethi K, Sarkar S, Das S, Mohanty B, Mandal M. (2010). Biomarkers for the diagnosis of thyroid cáncer. J Exp Ther Oncol. 2010;8(4):341-52.
Sosa JA, HannaJW, Robinson KA, Lanman RB. 2013. Increases in thyroid nodule fine-needle aspirations, operations, and diagnoses of thyroid cáncer in the United States. Surgery 154:1420-1426; discussion 1426-1427.
Qiu J, Zhang W, Zang C, LiuX., Liu F., Ge R, Sun Y., Xia Q. (2018). Identification of key genes and miRNAs markers of papillary thyroid cáncer. Biol Res. 2018 Nov 10;51(1):45).
Quang T., Eun Joo Lee, Melinda Gingman Huang, Young In Park, Aashish Khullar, Raymond A. Plodkowsk (2015) Diagnosis and Treatment of Patients with Thyroid Cáncer. Am Health Drug Benefits. 2015 Feb; 8(1): 30-40.
Xing M, Haugen BR, Schlumberger M. (2013). Progress in molecular-based management of 5 differentiated thyroid cáncer. The Lancet. Volume 381, Issue 9871,23-29 March2013, Pages 1058-1069)

## Claims

1. *In vitro* method for the diagnosis and prediction of thyroid cancer aggressiveness possibilities and type of precision surgery for tumor removal in a subject **CHARACTERIZED in that** said method comprises:
a) obtaining a thyroid tissue sample from the subject;
b) determining the level of expression of gene products in a sample of thyroid tissue obtained from the subject, wherein at least one or more of the gene products are expressed by at least one or more of the genes PTEN, BRAF, CD80, CTLA4, VEGFC, miR-16, miR-146b, miR-155, miR-181d, RNU6B and RET;
c) identify the sample of thyroid tissue as metastatic cancerous and non-metastatic cancerous, correlating the levels of expression of the genes determined in (b) with the presence or absence of lymph node metastasis in the tissue sample previously diagnosed with thyroid cancer, wherein the correlation is performed using the data from expression of the genes indicated in (b) from a plurality of tissue samples metastatic cancerous and non-metastatic cancerous tissue;
d) identifying the cancerous thyroid tissue sample as metastatic or non-metastatic with:
A sensitivity greater than or equal to 69% or greater than or equal to 90%;
A specificity greater than or equal to 78% or greater than or equal to 93%;
A positive predictive value greater than or equal to 52% or greater than or equal to 76%;
A negative predictive value greater than or equal to 55% or greater than or equal to 93%;
A positive probability ratio greater than or equal to 2 or greater than or equal to 12;
A positive post-test probability greater than or equal to 58% or greater than or equal to 90% A negative probability ratio greater than or equal to 0.19 or greater than or equal to 0.58; and
A negative post-test probability greater than or equal to 9% or greater than or equal to 32%; and
e) surgically removing the entire tumor from the subject with cervical dissection of lymph nodes or only a complete tumor removal or a portion of it if this cancerous thyroid tissue is identified as metastatic.

2. *In vitro* method for diagnosis and prediction of thyroid cancer aggressiveness and possibilities and type of precision surgery for tumor removal in a subject according to claim 1 **CHARACTERIZED in that** the correlation described in c) comprises the comparing the levels of expression of the genes indicated in b) with a (i) plurality of non-metastatic cancerous thyroid tissue samples and (ii) a plurality of samples of metastatic cancerous tissue.

3. *In vitro* method for diagnosis and prediction of thyroid cancer aggressiveness and possibilities and type of precision surgery for tumor removal in a subject according to claims 1 and 2 **CHARACTERIZED in that** the thyroid tissue sample cancer is identified as metastatic if there are different levels of expression in the gene products of cancerous thyroid tissue sample and gene expression data of (i) or if there are no significant differences in the levels of expression of the products between the sample of cancerous thyroid tissue and the gene expression data of (ii).

4. *In vitro* method for the diagnosis and prediction of thyroid cancer aggressiveness and possibilities and type of precision surgery for tumor removal in a subject according to claim 3 **CHARACTERIZED in that** the sample of cancerous thyroid tissue identifies as metastatic with:
A sensitivity greater than or equal to 69% or greater than or equal to 90%;
A specificity greater than or equal to 78% or greater than or equal to 93%;
A positive predictive value greater than or equal to 52% or greater than or equal to 76%;
A negative predictive value greater than or equal to 55% or greater than or equal to 93%;
A positive probability ratio greater than or equal to 2 or greater than or equal to 12;
A positive post-test probability greater than or equal to 58% or greater than or equal to 90% a negative probability ratio greater than or equal to 0.19 or greater than or equal to 0.58; and a negative post-test probability greater than or equal to 9% or greater than or equal to 32%.

5. *In vitro* method for the diagnosis and prediction of thyroid cancer aggressiveness possibilities and type of precision surgery for tumor removal in a subject **CHARACTERIZED in that** said method comprises:
a) obtaining a thyroid tissue sample from the subject,
b) determining the level of expression of gene products in a sample of cancerous thyroid tissue obtained from the subject, wherein at least one or more of the gene products are expressed by one or more of the genes PTEN, BRAF, CD80, CTLA4, VEGFC, miR-16, miR-146b, miR-155, miR-181d, RNU6B and RET,
c) identify lymph node metastasis in the subject previously diagnosed with papillary thyroid cancer, using a trained classification algorithm to classify samples of cancerous thyroid tissue based on the integration of the expression data of said products genes in two groups identified as:
(i) gene expression data of each sample of cancerous thyroid tissue are preprocessed, where ratios are generated between gene levels of expression of the gene products in step (b) and gene levels of expression of reference gene products, expansion of each of the variables is generated and dimensions of such variables are reduced; and
d) wherein,
(i) the pre-processed data for each cancerous thyroid tissue sample referred to in step (c)(i) are integrated into a classifier algorithm to classify the sample as metastatic or non-metastatic; wherein the algorithm was trained with expression levels preprocessing said gene products into a plurality of known samples with and without lymph node metastasis; wherein the method further comprises surgical removal complete tumor of subject with cervical dissection of lymph nodes or only one complete removal of the tumor or a portion thereof.

6. *In vitro* method for the diagnosis and prediction of thyroid cancer aggressiveness and possibilities and type of precision surgery for tumor removal in a subject according to claim 5 **CHARACTERIZED in that** the classification output score which grants the algorithm to the tissue samples to be analyzed in steps (c)(i) and (d)(i) are classified according to the classification cut-off, greater than or equal to 0.47, for reporting the probability of metastasis or non-metastasis in cancerous thyroid tissue of a subject.

7. *In vitro* method for the diagnosis and prediction OF thyroid cancer aggressiveness and the possibility and type of precision surgery for tumor removal in a subject according to claims 1 to 6 **CHARACTERIZED in that** the classifier algorithm comprises a multi-layer perceptron neural network analysis.

8. *In vitro* method for the diagnosis and prediction of thyroid cancer aggressiveness and possibilities and type of precision surgery for tumor removal in a subject according to claims 1 to 7 **CHARACTERIZED in that** the thyroid tissue samples are obtained by needle aspiration, fine needle aspiration, core needle biopsy, vacuum assisted biopsy, large core biopsy, incision biopsy, excision biopsy, or puncture biopsy with hollow punch.

9. *In vitro* method for the diagnosis and prediction of thyroid cancer aggressiveness and possibilities and type of precision surgery for tumor removal in a subject according with claims 1 to 8 **CHARACTERIZED in that** the gene products in analysis corresponds to RNA.

10. *In vitro* method for the diagnosis and prediction of thyroid cancer aggressiveness and possibilities and type of precision surgery for tumor removal in a patient according to claim 9 **CHARACTERIZED in that** the gene products under analysis correspond to RNA, wherein RNA is mRNA, rRNA, tRNA, or miRNA.

11. *In vitro* method for the diagnosis and prediction of thyroid cancer aggressiveness and possibilities and type of precision surgery for the tumor removal in a patient according to claim 10, **CHARACTERIZED in that** it comprises the determination of levels of RNA expression by microarray techniques, serial analysis of the gene expression (SAGE), transfer techniques, real-time PCR, quantitative PCR, semi-quantitative PCR, molecular bar coding techniques, Transcript Analysis with the aid of Affinity Capture (TRAC), PCR arrays, quantitative PCR in multiple configuration or quantitative nuclease protection assay (qNPA).

12. *In vitro* method for the diagnosis and prediction of thyroid cancer aggressiveness and possibilities and type of precision surgery for tumor removal in a subject according with claims 1 to 8 **CHARACTERIZED in that** the gene products in analysis corresponds to proteins.

13. *In vitro* method for the diagnosis and prediction of thyroid cancer aggressiveness and the possibilities and type of precision surgery for tumor removal in a patient according to claim 12, **CHARACTERIZED in that** it comprises the determination of protein expression of the gene products by ELISA techniques, mass spectrophotometry, transfer techniques, proteomics or immunohistochemistry techniques.

14. *In vitro* method for the diagnosis and prediction of thyroid cancer aggressiveness and the possibilities and type of precision surgery for tumor removal in a patient according to claims 1 to 13, **CHARACTERIZED in that** it comprises performing additionally an orthology analysis on a sample of thyroid tissue removed from a subject in step (a) to obtain a preliminary diagnosis.

15. *In vitro* method for diagnosis and prediction of thyroid cancer aggressiveness and the possibilities and type of precision surgery for tumor removal in a patient according to claims 1 to 14 **CHARACTERIZED in that** the samples with preliminary cancerous diagnoses are further analyzed by steps (b) and (c).

16. Kit for the diagnosis and prediction of thyroid cancer aggressiveness and possibilities and type of precision surgery for tumor removal in a subject **CHARACTERIZED in that** it comprises one or more reagents for the detection of one or more gene products, wherein the gene products are expressed by one or more genes corresponding to genes PTEN, BRAF, CD80, CTLA4, VEGFC, miR-16, miR-146b, miR-155, miR-181d, RNU6B and RET.

17. Kit for the diagnosis and prediction of the thyroid cancer aggressiveness and possibilities and type of precision surgery for tumor removal in a subject according to claim 16 **CHARACTERIZED IN that** the reagents correspond to antibodies, wherein each antibody specifically binds a gene product of polypeptides or proteins.

18. Kit for the diagnosis and prediction of thyroid cancer aggressiveness and possibilities and type of precision surgery for tumor removal in a subject according to the claims 16 and 17 **CHARACTERIZED in that** the reagents correspond to oligonucleotides or a set of oligonucleotides which bind specifically to a nucleic acid gene product.

19. Kit for the diagnosis and prediction of thyroid cancer aggressiveness and possibilities and type of precision surgery for tumor removal in a subject according to the claim 18 **CHARACTERIZED in that** the kit comprises reagents for performing PCR assay.

20. Kit for the diagnosis and prediction of thyroid cancer aggressiveness and possibilities and type of precision surgery for tumor removal in a subject according to the claims 18 and 19 **CHARACTERIZED in that** the reagents comprise agents further selected from the group of a thermostable polymerase, a mixture of deoxynucleotides and a detectably labeled probe.

21. Kit for the diagnosis and prediction of thyroid cancer aggressiveness and possibilities and type of precision surgery for tumor removal in a subject according to the claims 18 to 20 **CHARACTERIZED in that** the labeled probe detectably comprises a fluorophore.

22. Kit for the diagnosis and prediction of thyroid cancer aggressiveness and possibilities and type of precision surgery for tumor removal in a subject according to the claims 16 to 21 **CHARACTERIZED in that** the reagents are each attached to a substrate, particularly covalently bonded.

23. Kit for the diagnosis and prediction of thyroid cancer aggressiveness and possibilities and type of precision surgery for tumor removal in a subject according to the claims 16 to 22 **CHARACTERIZED in that** the reagents are attached to different regions of a solid array, particularly a microarray.

24. Kit for the diagnosis and prediction of the thyroid cancer aggressiveness and possibilities and type of precision surgery for the tumor removal in a subject according to the claims 16 to 23, **CHARACTERIZED in that** it determines the levels of expression of one or more gene products expressed by the genes PTEN, BRAF, CD80, CTLA4, VEGFC, miR-16, miR-146b, miR-155, miR-181d, RNU6B and RET.

25. Kit for the diagnosis and prediction of thyroid cancer aggressiveness and possibilities and type of precision surgery for tumor removal in a subject according to claim 24 **CHARACTERIZED in that** the determination of the expression levels of one or more genes allows identifying the presence or absence of cancer in the sample of thyroid tissue.

26. Kit for the diagnosis and prediction of thyroid cancer aggressiveness and possibilities and type of precision surgery for tumor removal in a subject according to claims 24 and 25 **CHARACTERIZED in that** the levels of expression of one or more genes identified in a sample of thyroid tissue are associated by comparing the level of expression with a normal control of each gene product.

27. Kit for the diagnosis and prediction of the thyroid cancer aggressiveness and possibilities and type of precision surgery for tumor removal in a subject according to claims 24 to 26 **CHARACTERIZED in that** the sample of thyroid tissue is identified as cancerous if there is a difference in the levels of expression of one or more genes with respect to the levels of expression of one or more genes of the samples used as a control.

28. Kit for the diagnosis and prediction of the thyroid cancer aggressiveness and possibilities and type of precision surgery for the tumor removal in a subject according to the claim 24 to 27, **CHARACTERIZED in that** the identification of cancerous tissue comprises comparing levels of expression of one or more gene products in two sets of of biological samples:
i) multiple samples of normal thyroid tissue and;
ii) multiple samples of cancerous tissue
where if there are differences between the levels of expression of one or more gene products described in (i) and (ii) the sample is considered cancerous.

29. Kit for the diagnosis and prediction of thyroid cancer aggressiveness and possibilities and type of precision surgery for tumor removal in a subject according to the claim 28 **CHARACTERIZED in that** the gene product corresponds to DNA, RNA or proteins.

30. Kit for the diagnosis and prediction of the thyroid cancer aggressiveness and possibilities and type of precision surgery for the tumor removal in a subject according to the claim 29 **CHARACTERIZED in that** the RNA gene product corresponds to mRNA.

31. Kit for the diagnosis and prediction of thyroid cancer aggressiveness and possibilities and type of precision surgery for tumor removal in a subject according to claim 30 **CHARACTERIZED in that** the quantification of levels of expression of mRNA from one or more of the genes PTEN, BRAF, CD80, CTLA4, VEGFC, miR-16, miR-146b, miR-155, miR-181d, RNU6B and RET is performed by microarray techniques, Serial Analysis of Gene Expression (SAGE), transfer techniques, real-time PCR, quantitative PCR, semi-quantitative PCR, molecular bar coding techniques, Transcript Analysis with the aid of Affinity Capture (TRAC), PCR arrays, quantitative PCR in multiple configuration or quantitative nuclease protection assay (qNPA).

32. Kit for the diagnosis and prediction of thyroid cancer aggressiveness and possibilities and type of precision surgery for tumor removal in a subject according to the claim 28 **CHARACTERIZED in that** the gene product corresponds to polypeptides or proteins.

33. Kit for the diagnosis and prediction of the thyroid cancer aggressiveness and the possibilities and type of precision surgery for the tumor removal in a subject according to claim 32 **CHARACTERIZED in that** the quantification of the levels of expression of the proteins of at least one or more genes of the genes PTEN, BRAF, CD80, CTLA4, VEGFC, miR-16, miR-146b, miR-155, miR-181d, RNU6B and RET are performed by ELISA techniques, mass spectrophotometry, transfer techniques, proteomics or immunohistochemistry techniques.

34. Reagents for the diagnosis and prediction of thyroid cancer aggressiveness and possibilities and type of precision surgery for tumor removal in a subject **CHARACTERIZED in that** they detect the levels of expression of one or more gene products corresponding to the genes PTEN, BRAF, CD80, CTLA4, VEGFC, miR-16, miR-146b, miR-155, miR-181d, RNU6B and RET in a sample of thyroid tissue.

35. Reagents for the diagnosis and prediction of thyroid cancer aggressiveness and possibilities and type of precision surgery for tumor removal in a subject according to the claim 34 **CHARACTERIZED in that** they allow the identification of the levels of expression of one or more genes in a thyroid tissue sample, wherein the reagents comprise:
i) antibodies specifically binding to one or more gene products; or
ii) oligonucleotides or set of oligonucleotides that specifically bind acids nucleic acids of one or more gene products.

36. Reagents for the diagnosis and prediction of thyroid cancer aggressiveness and possibilities and type of precision surgery for tumor removal in a subject according to the claims 34 and 35 **CHARACTERIZED in that** they comprise:
i) one or more reagents for processing a thyroid tissue sample; or
ii) one or more control gene products.

37. Use of the reagents according to claims 34 to 36 **CHARACTERIZED in that** they serve in the diagnosis and prediction of thyroid cancer aggressiveness and the possibilities and type of precision surgery for tumor removal in a subject.

38. Use of predictive markers of thyroid cancer aggression and possibilities and type of precision surgery for tumor removal in a subject **CHARACTERIZED in that** it comprises
a) amplifying and detecting using a molecular biology method such as PCR and ELISA, using one or more of the markers PTEN, BRAF, CD80, CTLA4, VEGFC, miR-16, miR-146b, miR-155, miR-181d, RNU6B and RET;
b) quantifying overexpression or underexpression of one or more of the PTEN markers, BRAF, CD80, CTLA4, VEGFC, miR-16, miR-146b, miR-155, miR-181d, RNU6B, and RET; and
c) correlating the quantification of the overexpression or underexpression obtained with the prediction of thyroid cancer aggressiveness and the possibilities and type of thyroid precision for tumor removal in a subject,
(i) wherein the cancerous thyroid tissue sample is identified as metastatic or non-metastatic with:
A sensitivity greater than or equal to 69% or greater than or equal to 90%;
A specificity greater than or equal to 78% or greater than or equal to 93%;
A positive predictive value greater than or equal to 52% or greater than or equal to 76%;
A negative predictive value greater than or equal to 55% or greater than or equal to 93%;
A positive probability ratio greater than or equal to 2 or greater than or equal to 12;
A positive post-test probability greater than or equal to 58% or greater than or equal to 90% A negative probability ratio greater than or equal to 0.19 or greater than or equal to 0.58; and
A negative post-test probability greater than or equal to 9% or greater than or equal to 32%.
(ii) wherein, the results of the method further comprise surgical removal complete tumor of subject with cervical dissection of lymph nodes or only one complete removal of the tumor or a portion thereof.

39. Use of predictive markers of thyroid cancer aggression and possibilities and type of precision surgery for tumor removal in a subject according to the claim 38 **CHARACTERIZED in that** the gene products under analysis correspond to nucleic acids.

40. Use of predictive markers of thyroid cancer aggressiveness and possibilities and type of precision surgery for tumor removal in a subject according to the claim 39 **CHARACTERIZED in that** the gene products under analysis correspond a RNA, wherein RNA is mRNA, rRNA, tRNA or miRNA.

41. Use of the predictive markers of thyroid cancer aggressiveness and the possibilities and type of precision surgery for tumor removal in a subject according to claims 39 to 40 **CHARACTERIZED in that** it comprises the determination of RNA levels of expression by microarray techniques, serial analysis of gene expression (SAGE), transfer techniques, real-time PCR, quantitative PCR, semi-quantitative PCR, molecular bar coding techniques, Transcript Analysis with the aid of Affinity Capture (TRAC), PCR arrays, quantitative PCR in multiple configuration or quantitative nuclease protection assay (qNPA).

42. Use of predictive markers of thyroid cancer aggressiveness and the possibilities and type of precision surgery for tumor removal in a subject according to
claim 38 **CHARACTERIZED in that** the gene products under analysis correspond to proteins.

43. Use of the predictive markers of thyroid cancer aggressiveness and the possibilities and type of precision surgery for tumor removal in a subject according to claim 42, **CHARACTERIZED in that** it comprises the determination of protein expression of the gene products by ELISA techniques, mass spectrophotometry, transfer techniques, proteomics or immunohistochemistry techniques.
